# EUROPÄISCHE PATENTSCHRIFT

(11) **EP 2 791 086 B1**
(45) Veröffentlichungstag und Bekanntmachung des Hinweises auf die Patenterteilung: **14.11.2018**
(21) Anmeldenummer: 12806350.0
(22) Anmeldetag: 13.12.2012
(51) Int. Cl.: C07C 1/20, C07C 9/15, C07C 9/16, C07C 29/34, C07C 31/125, C07C 45/45, C07C 49/04, C10L 1/02, C10L 1/04, C10G 3/00

(54) **VERFAHREN ZUR KATALYTISCHEN KONDENSATION ODER KOPPLUNG**
METHOD FOR THE PURPOSE OF A CATALYTIC CONDENSATION OR COUPLING
PROCÉDÉ SERVANT À RÉALISER UN COUPLAGE OU UNE CONDENSATION CATALYTIQUE

(30) Priorität: 14.12.2011 DE 102011120923; 15.12.2011 DE 102011121087
(43) Veröffentlichungstag der Anmeldung: 22.10.2014
(73) Patentinhaber: Fraunhofer Gesellschaft zur Förderung der angewandten Forschung e.V., 80686 München (DE); SASOL Germany GmbH, 20537 Hamburg (DE)
(72) Erfinder: KRAFT, Axel, 45759 Oer-Erkenschwick (DE); MENNE, Andreas, 45470 Mülheim an der Ruhr (DE); BREITKREUZ, Klaas, 44879 Bochum (DE); GROSS, Thoralf, 25541 Brunsbüttel (DE); ZIEHE, Holger, 25524 Itzehoe (DE)
(86) Internationale Anmeldenummer: PCT/EP2012/005152
(87) Internationale Veröffentlichungsnummer: WO 2013/087211

(56) Entgegenhaltungen:
- EP-A2- 0 299 720
- WO-A1-2011/157322
- OLSON ET AL: "Higher- Alcohols Biorefinery-Improvement of Catalyst for Ethanol Conversion", APPLIED BIOCHEMISTRY AND BIOTECHNOLOGY, HUMANA PRESS, INC, UNITED STATES, Bd. 113-116, 1. Januar 2004 (2004-01-01), Seiten 913-932, XP009152756, ISSN: 0273-2289, DOI: 10.1385/ABAB:115:1-3:0913
- THOMAS J. BRUNO ET AL: "Comparison of Biomass-Derived Turbine Fuels with the Composition-Explicit Distillation Curve Method", ENERGY & FUELS, Bd. 25, Nr. 4, 6. März 2011 (2011-03-06), Seiten 1847-1858, XP055058875, ISSN: 0887-0624, DOI: 10.1021/ef200115b

## Beschreibung

Die vorliegende Erfindung betrifft das technische Gebiet der katalytischen Kondensation bzw. Kopplung von Alkoholen oder Carbonylverbindungen mit CH-aciden Verbindungen, insbesondere zur Herstellung höherer Alkohole, Aldehyde, Ketone, Aromaten und/oder Alkane sowie deren Gemischen. Insbesondere betrifft die vorliegende Erfindung ein Verfahren zur katalytischen Kondensation bzw. Kopplung von Oxo- und/oder Hydroxylfunktionen enthaltenden organischen Verbindungen mit CH-aciden Verbindungen.

Weiterhin betrifft die vorliegende Offenbarung Produkte und Produktgemische, insbesondere Alkohole, Aldehyde, Ketone, Alkane und/oder Aromaten sowie deren Gemische, welche durch das erfindungsgemäße Verfahren erhältlich sind.

Darüber hinaus betrifft die vorliegende Offenbarung die Verwendung der offenbarten Produkte oder Produktgemische als Brenn- bzw. Kraftstoffe sowie als Chemikalien für private und industrielle Zwecke.

Schließlich betrifft die vorliegende Offenbarung die Verwendung eines mit mindestens einem Metall versehenen Aktivkohlesubstrats als Katalysator für die katalytische Kondensation bzw. Kopplung von Oxo- und/oder Hydroxylfunktionen enthaltenden organischen Verbindungen mit CH-aciden Verbindungen.

Ein zentrales Problem der chemisch-industriellen Produktion ist die Synthese längerkettiger nichtpolymerer Verbindungen ausgehend von kurzkettigen, kostengünstig sowie im industriellen Maßstab verfügbaren Ausgangstoffen zu Zwecken der Herstellung hochwertiger chemischer Produkte, wie beispielsweise Tenside, Additive oder auch bestimmter Brennstofffraktionen. In diesem Zusammenhang sind insbesondere höhermolekulare Alkohole von Bedeutung.

Eine Möglichkeit beispielsweise höhermolekulare Alkohole zu erzeugen, ist die sogenannte Guerbet-Reaktion, bei welcher primäre oder sekundäre Alkohole insbesondere in basischem Milieu gekoppelt und zumeist verzweigte primäre Alkohole erhalten werden.

Bei der nach dem Chemiker Marcel Guerbet benannten Reaktion im eigentlichen Sinne handelt es sich um eine organische Reaktion, bei welcher primäre oder sekundäre Alkohole katalytisch unter Abspaltung eines Äquivalents Wasser zu beta-alkylierten dimerisierten Alkoholen umgesetzt werden, wobei üblicherweise an sich bekannt Hydrierkatalysatoren, wie beispielsweise sogenanntes Raney-Nickel, oder Alkalimetallhydroxide oder -alkoxide eingesetzt werden.

Die Herstellung dieser sogenannten Guerbet-Alkohole erfolgt nach dem Stand der Technik üblicherweise in einem homogen-katalytischen Prozess, bei welchem vornehmlich Alkali- und Erdalkali-Katalysatoren in Form ihrer Hydroxide zum Einsatz kommen, welche im Anschluss an die Synthese wieder aufwendig abgetrennt werden müssen und damit Abfall erzeugen. Dabei verläuft die Synthese unter erhöhtem Druck und bei Temperaturen, welche üblicherweise unterhalb des Siedepunktes der Ausgangsmaterialien liegen, was die Flexibilität des Verfahrens für niedrig siedende Alkohole sehr stark einschränkt (vgl. DE 693 16 349 T2, EP 0 299 720 A2, US 766 677 A, US 2010/0298613 A1, WO 91/04242 A1 und WO 2011/054483 A1).

Aus diesen höheren Alkoholen können in nachfolgenden Reaktionsschritten weitere Produkte, wie beispielsweise Alkane, hergestellt werden. Insbesondere ist es zudem in aufwendigen Prozessen möglich, Alkangemische mit vergleichbaren Eigenschaften wie Flugbenzin, insbesondere Kerosin, zu erhalten.

Aufgrund des enormen Bedarfs an Flugzeugkerosin, sogenanntem JET-A1 bzw. Jetfuel, von weltweit etwa 200 Millionen Tonnen - in Deutschland alleine wurden im Jahr 2010 über 8,5 Mio. Tonnen verbraucht - ist für die Bereitstellung der benötigten Alkane auf biogener Basis die Umwandlung eines breit verfügbaren, günstigen und regenerativen Rohstoffs erforderlich. Auch insbesondere vor dem Hintergrund des zunehmenden Handels mit CO₂-Zertifikaten ist die Herstellung eines nachhaltigen Biokerosins bzw. dessen Beimischung zu fossil hergestelltem Kerosin für die Luftfahrtindustrie von sehr großem Interesse.

Üblicherweise werden die als Luftfahrtbetriebsstoffe eingesetzten Paraffine in Form eines sogenannten Mitteldestillats der Erdölraffination gewonnen und bestehen unter anderem zu je ca. 35 Massenprozent aus verzweigten und unverzweigten C₈-C₁₅-Alkanen.

Bekannte Prozesse, Kerosin auf Basis nachwachsender Rohstoffe herzustellen, basieren hauptsächlich auf der Hydrierung oder dem Hydrotreating von Pflanzenölen. Dabei entstehen jedoch hauptsächlich unverzweigte und gesättigte Alkane, deren Siede- und Gefrierpunktsbereich deutlich von fossilbasiertem Kerosin abweicht. Daher sind zusätzliche Isomerisierungs- und Hydrocrackingschritte notwendig.

Bislang existiert noch kein Verfahren, um Kerosin durch direkte katalytische Kondensation aus Alkoholen herzustellen, insbesondere nicht auf Basis nachwachsender Rohstoffe.

Weiterhin können höhere Alkohole, insbesondere verzweigte Alkohole (sogenannte Guerbet-Alkohole) und lineare Alkohole, sowie Alkane und Alkene im Siedebereich von Jetfuel bisher nicht durch eine einstufige heterogenkatalytische Kondensation ohne Wasserstoff aus insbesondere biobasierten kürzerkettigen Alkoholen hergestellt werden. Es muss daher z. B. eine Dehydratisierung zu Alkenen und Oligomerisierung der Alkene vorausgeschaltet werden, um so die benötigte Kohlenstoffkette aufzubauen. Um Kerosin zu erzeugen, muss dabei nachträglich hydriert bzw. zur Herstellung von Alkoholen wieder hydratisiert werden (vgl. insbesondere EP 0 099 690 A2).

Nach dem Stand der Technik können höhere Alkohole gleichermaßen durch Aldolkondensation hergestellt werden, wobei homogene Katalysatoren, wie anorganische Hydroxide oder ähnlich starke Basen, eingesetzt werden. Dieser Weg ist allerdings bisher nicht wirtschaftlich für die Kerosinherstellung nutzbar.

Die mehrstufige Herstellung von biobasiertem Kerosin ist auch aus biobasierten Furfuralen bzw. deren Derivaten und Ketonen, wie z. B. Aceton, möglich. Die hierfür benötigten Furfurale müssen allerdings erst aufwendig aus Lignocellulose gewonnen werden.

In weiteren Verfahren zur Herstellung von Kerosin nach dem Stand der Technik werden zum Teil sauerstoffhaltige Verbindungen eingesetzt, so dass die erhaltenen Produkte ebenfalls nicht der gültigen Norm für Kerosin bzw. Flugzeugkraftstoffen, insbesondere Jetfuel, entsprechen. Ein derartiges Verfahren wird beispielsweise in der EP 1 218 472 A1 beschrieben.

Weiterhin ist die Herstellung von Flugbenzin über die Dehydratisierung von Isobutanol und anderen durch Fermentation hergestellten Alkoholen, insbesondere den sogenannten Fuselalkoholen, sowie der darauf folgenden Oligomerisierung der entstehenden Alkene mit nachfolgender Hydrierung bekannt, wie beispielsweise im IATA 2010 Report on Alternative Fuels (Dezember 2010), Ref. No: 9709-03, ISBN 978-92-9233-491-8, ausgeführt wird.

Da die Kondensation insbesondere von Alkoholen und Aldehyden zu höheren Alkoholen, Alkanen etc. und gegebenenfalls deren weitere Umsetzung zumindest in der Theorie einen gangbaren Zugang zu höhermolekularen Verbindungen verspricht, hat es im Stand der Technik nicht an Versuchen gefehlt, die bestehenden Kondensationsverfahren zu verbessern bzw. neue Verfahren auf dieser Basis zu entwickeln.

Die CA 2 298 545 A1 betrifft ein Verfahren zur Herstellung metallfreier Guerbet-Alkohole, wobei primäre bzw. sekundäre Alkohole in Gegenwart von alkalischen Katalysatoren bzw. Schwermetallkatalysatoren bei hohen Temperaturen unter Entfernung des entstehenden Wassers kondensiert werden.

Die DE-OS 29 12 068 betrifft ein Verfahren zur Herstellung von Kohlenwasserstoffen, bei welchem Alkohole mittels Übergangs- bzw. Schwermetallkatalysatoren umgesetzt werden. Bei dem geschilderten Verfahren werden vorrangig Olefine sowie in geringerem Maße Aromaten als Produkte erhalten. Weiterhin betrifft die EP 1 052 234 A1 ein Verfahren zur Herstellung von Ausgangsmaterialien bzw. Rohstoffen für die chemische Industrie sowie von hochoktanigen Treibstoffen durch katalytische Umsetzung von Ethanol mittels eines Calciumphosphatkatalysators, welcher ein aktivierendes Metall enthält.

Die wissenschaftliche Veröffentlichung "Synthesis of Biogasoline from Ethanol over Hydroxyapatite Catalysts", T. Tsuchida, T. Yoshioka, S. Sakuma, T. Takeguchi und W. Ueda, Ind. Eng. Chem. Res. 47, Seiten 1443 bis 1452 (2008), betrifft die Herstellung von biobasierten Treibstoffen aus Ethanol.

Weiterhin betrifft die wissenschaftliche Publikation "Integration of C-C coupling reactions of biomass-derived oxygenates to fuel-grade compounds", E. I. Gürbüz, E. L. Kunkes und J. A. Dumesic, Applied Catalysis B: Environmental 94, Seiten 134 bis 141 (2010), die Umsetzung von aus biologischen Prozessen erhaltenen sauerstoffhaltigen organischen Verbindungen zu Treibstoffen.

In ähnlicher Weise betrifft die wissenschaftliche Veröffentlichung "Conversion of biomass-derived butanal into gasoline-range branched hydrocarbon over Pd-supported catalysts", S. M. Kim, M. E. Lee, J.-W. Choi, D. J. Suh und Y.-W. Suh, Catalysis Communications 16, Seiten 108 bis 113 (2011), die Umsetzung von mittels Biosynthese erhaltenem Butanal zu Treibstoffen.

Darüber hinaus betrifft die wissenschaftliche Veröffentlichung "Combined solid base/hydrogenation catalysts for industrial condensation reactions", F. King und G. J. Kelly, Catalysis Today 73, Seiten 75 bis 81 (2002), Katalysatoren für industrielle angewendete Kondensationsreaktionen.

Die wissenschaftliche Publikation "Reactions of methanol and higher alcohols over H-ZSM-5", A. C. Gujar, V. K. Guda, M. Nolan, Q. Yan, H. Toghiani und M. G. White, Applied Catalysis A: General 363, Seiten 115 bis 121 (2009), betrifft Kondensationsreaktionen von Methanol und höheren Alkoholen.

Die EP 0 299 720 A2 betrifft ein Verfahren zur Kondensation von Alkoholen, bei welchem verzweigte Alkohole mittels Guerbet-Kondensation aus primären C₁ bis C₂₀-Alkoholen in Gegenwart eines Katalysators, welcher aus fein verteilt auf eine Aktivkohlenoberfläche aufgebrachtem elementaren Platin besteht, und Kalium- oder Natrium-hydroxid erhalten werden. Es hat sich gezeigt, dass kleine Mengen des Katalysators effektiv im Temperaturbereich von 175 °C bis 210 °C verwendet werden können, wobei Umsatzraten von etwa 85 % bei ein- bis fünfstündigen Reaktionszeiten erhalten werden.

*Die wissenschaftliche Publikation* Olson, E. S. et al., Higher-Alcohols Biorefinery, Applied Biochemistry and Biotechnology, 113 bis 116, 2004, Seiten 913 bis 932*,* betrifft die Herstellung höherer Alkohole unter Verwendung von Aktivkohlekatalysatoren.

Weiterhin betrifft die wissenschaftlichen Veröffentlichung T.J. Bruno und E. Baibourine, Comparison of Biomass-Derived Turbine Fuels with the Composition-Explicit Distillation Curve Method, Energy Fuels, 25, 2011, Seiten 1847 bis 1858, den Vergleich von Standardflugbenzin JP-8 mit Flugtreibstoffen aus natürlichen Quellen. Insbesondere werden die Destillationskurven der jeweiligen Gemische miteinander verglichen.

Die WO 2011/157322 A1 betrifft ein Verfahren zur Herstellung höherer Alkohol und/oder Aldehyde sowie deren Gemische durch katalytische Umsetzung von Ethanol, wobei die Umsetzung in Gegenwart mindestens eines Katalysators erfolgt, wobei der Katalysator ein Aktivkohlesubstrat, welches mit mindestens einem Metall versehen ist, insbesondere mindestens eine Metalldotierung aufweist, umfasst.

Schließlich beschreibt die wissenschaftliche Publikation "Hydrotalcidederived mixed oxides as catalyst for different C-C bond formation reactions from bioorganic materials", S. Ordóñez, E. Diaz, M. León und L. Faba, Catalysis Today, Vol. 167, Seiten 71 bis 76 (2011), einerseits die Selbstkondensation von Aceton sowie die Selbstkondensation von Ethanol jeweils in der Gasphase und andererseits die Aldolkondensation von Furfurylalkohol mit Aceton in der flüssigen Phase. Die Kondensationsreaktionen von Aceton bzw. Ethanol in der Gasphase können lediglich in stark verdünnten Gasströmen durchgeführt werden und liefern äußerst geringe Umsatzraten. Auch die Flüssigphasenumsetzung von Furfurylalkohol und Aceton kann nur mit äußerst geringen Eduktkonzentrationen durchgeführt werden und benötigt mindestens 24 Stunden Reaktionszeit, um Umsatzraten von ca. 70 % zu erzielen, wobei die Produktselektivität äußerst gering ist.

Die zuvor beschriebenen Verfahren eignen sich folglich nicht zur Darstellung chemischer Verbindungen, insbesondere nicht im industriellen Maßstab.

Die zuvor geschilderten Verfahren des Standes der Technik besitzen allesamt den Nachteil, dass Kondensationsreaktion von Alkoholen und Carbonylverbindungen nur in geringen Ausbeuten, insbesondere nur in geringen Raum-Zeit-Ausbeuten bzw. nur mit niedrigen Raumgeschwindigkeiten, ablaufen, so dass diese Verfahren wenig effizient und nicht ökonomisch sinnvoll durchführbar sind.

Zudem ist bei den Verfahren des Standes der Technik eine aufwendig zu realisierende Prozessführung unter Verdünnung der Edukte bzw. Reaktanden mit Inertgas oder Lösemitteln, wie beispielsweise Wasser, erforderlich. Die meisten der zuvor beschriebenen Verfahren des Standes der Technik verwenden zudem Katalysatorsysteme mit unter großtechnischen Bedingungen unzureichenden Standzeiten der Katalysatoren. Auch ist es oftmals schwierig, kontrollierbare Reaktionsbedingungen zu schaffen, so dass nicht in verlässlicher Weise reproduzierbare Ausbeuten und Produktgemische erhalten werden können. Die meisten der beschriebenen Verfahren sind folglich für großtechnische Anwendungen nicht geeignet.

Die Aufgabe der vorliegenden Erfindung besteht somit darin, ein Verfahren bereitzustellen, welches eine effiziente Kondensation bzw. Kopplung von organischen Verbindungen auch unter industriellen Bedingungen ermöglicht und die zuvor geschilderten Nachteile des Standes der Technik zumindest im Wesentlichen vermeidet oder aber wenigstens verringert bzw. abschwächt.

Insbesondere ist eine Aufgabe der vorliegenden Erfindung darin zu sehen, ein Verfahren bereitzustellen, welches insbesondere zur Erzeugung höherer Alkohole, Aldehyde, Ketone, Aromaten und/oder Alkane sowie deren Gemischen geeignet ist und insbesondere auch unter industriellen bzw. großtechnischen Anwendungsbedingungen durchführbar ist.

Zur Lösung der zuvor geschilderten Aufgabe schlägt die vorliegende Erfindung ein Verfahren nach Anspruch 1 vor; weitere vorteilhafte Ausgestaltungen sind Gegenstand der diesbezüglichen abhängigen Ansprüche.

Es versteht sich von selbst, dass Ausgestaltungen, Ausführungsformen, Vorteile oder dergleichen, welche nachfolgend - zu Zwecken der Vermeidung von unnötigen Wiederholungen - nur zu einem Erfindungsaspekt ausgeführt werden, selbstverständlich auch in Bezug auf die übrigen Erfindungsaspekte entsprechend gelten.

Bei allen nachstehend genannten relativen bzw. prozentualen, insbesondere gewichtsbezogenen Mengenangaben, ist zudem zu beachten, dass diese im Rahmen der vorliegenden Erfindung vom Fachmann derart auszuwählen sind, dass in der Summe - gegebenenfalls unter Einbeziehung weiterer Komponenten bzw. Inhaltsstoffe bzw. Zusatzstoffe bzw. Bestandteile, insbesondere wie nachfolgend definiert - stets 100 % resultieren. Dies versteht sich für den Fachmann aber von selbst.

Zudem gilt, dass alle im Folgenden genannten Werte- bzw. Parameterangaben oder dergleichen grundsätzlich mit genormten bzw. standardisierten oder explizit angegebenen Bestimmungsverfahren oder aber mit dem Fachmann auf diesem Gebiet an sich geläufigen Bestimmungsmethoden ermittelt bzw. bestimmt werden können.

Dies vorausgeschickt, wird im Folgenden die vorliegende Erfindung näher beschrieben.

Gegenstand der vorliegenden Erfindung - gemäß einem **ersten** Aspekt der vorliegenden Erfindung - ist somit ein Verfahren zur katalytischen Kondensation bzw. Kopplung von mindestens eine Oxo- und/oder Hydroxylfunktion enthaltenden organischen Verbindungen mit CH-aciden Verbindungen, mit welchem insbesondere höhere Alkohole, Aldehyde, Ketone, Aromaten und/oder Alkane sowie deren Gemische erzeugt werde können.

Das erfindungsgemäße Verfahren wird derart durchgeführt, dass als Eduktgemisch
(A) mindestens eine mindestens eine Oxo- und/oder Hydroxylfunktion enthaltende organische Verbindung, welche mindestens 3 Kohlenstoffatome aufweist und ausgewählt ist aus der Gruppe von primären und/oder sekundären Alkoholen, Aldehyden und Ketonen sowie deren Mischungen,
   mit
(B) mindestens einer CH-aciden Verbindung, welche mindestens 3 Kohlenstoffatome aufweist, wobei die CH-acide Verbindung (B) ausgewählt ist aus der Gruppe von primären und/oder sekundären Alkoholen, Carbonsäuren, Carbonsäureanhydriden, Carbonsäureestern, Aldehyden, Ketonen, Nitrilen, Nitroverbindungen, organischen Nitraten und deren Mischungen,
wobei (A) und (B) identisch sein können,
eingesetzt und in Gegenwart mindestens eines Katalysators umgesetzt wird, wobei der Katalysator ein Aktivkohlesubstrat, welches mit mindestens einem Metall versehen ist, umfasst,
wobei als Katalysator eine Formaktivkohle zum Einsatz kommt, welche herstellbar ist durch ein Verfahren zur Herstellung von Formaktivkohle aus einem Kohlenstoffträger, einem Bindemittel und einer katalytischen Komponente der allgemeinen Formel (I)

[M]ₘ₃[AOₙ₄]ₘ₄ (I)

wobei
- M ein Kation bezeichnet und ausgewählt ist aus der Gruppe von Alkali- oder Erdalkalimetallkationen;
- m3 und m4 stöchiometrische Koeffizienten mit ganzen Zahlen mit m3 ≥ 1 und m4 ≥ 1 bezeichnen;
- [AOₙ₄] ein sauerstoffhaltiges Anion mit dem ganzzahligen stöchiometrischen Koeffizienten n4 ≥ 1 bezeichnet;
- [AOₙ₄] vorzugsweise ausgewählt wird aus der Gruppe von Carbonaten oder Hydroxiden,
wobei das Bindemittel erhalten wird aus der Umsetzung eines wasserlöslichen kohlenhydrathaltigen Ausgangsstoffes mit einem Glucosegehalt von ≥ 50 Gew.-%, wobei der Kohlenstoffträger zunächst mit der katalytischen Komponente vermischt wird, wobei anschließend die Mischung aus katalytischer Komponente und Kohlenstoffträger mit dem Bindemittel vermischt wird, wobei die so erhaltene Mischung aus Kohlenstoffträger, katalytischer Komponente und Bindemittel zu Formlingen verpresst wird und wobei die Formlinge carbonisiert und aktiviert werden, wobei das Bindemittel aus der Umsetzung des kohlenhydrathaltigen Ausgangsstoffes mit einem Zuschlagstoff erhalten wird, wobei zum Erhalt des Bindemittels der Zuschlagstoff dem kohlenhydrathaltigen Ausgangsstoff zugegeben wird vor der Vermischung des Bindemittels mit der Mischung aus dem Kohlenstoffträger und der katalytischen Komponente, wobei der Zuschlagstoff ausgewählt ist aus der Gruppe von Phosphorsäuren und/oder deren Salzen, Schwefelsäuren und/oder deren Salzen und/oder Schwefelsäurederivaten und/oder deren Salzen.

Das erfindungsgemäße Verfahren eignet sich in hervorragender Weise zur Herstellung von höheren Alkoholen, Aldehyden, Ketonen, Aromaten und/oder Alkanen sowie deren Gemischen in hohen Ausbeuten und mit hoher Selektivität. Dies ist umso überraschender, als zu der erfindungsgemäßen Verfahrensdurchfuhrung die Verwendung eines aktivkohlebasierten Katalysators genügt, welcher mit mindestens einem Metall beladen bzw. imprägniert ist.

Zusätzlich entstehen bei dem beschriebenen Prozess durch Kondensation von zwei oder mehreren linearen oder verzweigten Alkoholen bzw. Aldehyden insbesondere auch höhere, insbesondere in beta-Position verzweigte Alkohole - die sogenannten Guerbet-Alkohole - bzw. deren entsprechende Aldehyde.

In Abhängigkeit von den Prozessbedingungen bietet das erfindungsgemäße Verfahren insbesondere die Möglichkeit zur Herstellung von sogenannten Guerbet-Alkoholen in einem Festbettreaktor. Die gleichfalls als Nebenprodukt entstehenden Alkane können gegebenenfalls selektiv abgetrennt werden, so dass eine reine Alkoholfraktion einerseits und eine reine Alkanfraktion andererseits erhalten werden kann, sofern gewünscht (vgl. z. B. US 7 465 846 B2).

Überraschenderweise kann das erfindungsgemäße Verfahren jedoch auch derart durchgeführt werden, dass ein großer Anteil der Produkte als hydrierte Guerbet-Alkohole oder deren Umlagerungsprodukte, im wesentlichen Alkane und in geringen Mengen Alkene, anfällt. Durch die Zugabe von Wasser zu der Reaktionsmischung oder zu den Edukten kann die Selektivität hin zu Alkanen noch weiter erhöht werden. Die zusätzliche Einleitung von Wasserstoff während der Kopplungs- bzw. Kondensationsreaktion ist zwar optional, wird aber für die ablaufenden Hydrierprozesse nicht unbedingt benötigt.

Nicht vorhersehbar war gleichfalls, dass auch niedrig siedende Alkohole, wie beispielsweise n-Propanol, n-Butanol, n-Pentanol oder n-Hexanol, wahlweise ohne Anwendung von Druck, zu höheren Alkoholen, insbesondere Guerbet-Alkoholen, und optional - insbesondere ohne weitere Aufreinigung - zu Treibstoffen, wie Benzin oder Kerosin, umgesetzt werden können.

Als besonders vorteilhaft erweist es sich in diesem Fall, dass das erfindungsgemäße Verfahren kontinuierlich ausgeführt werden kann. Dies ist bei der klassischen Guerbet-Alkoholsynthese, die diskontinuierlich bzw. batchweise betrieben wird, nicht möglich, so dass deren Produkte schon allein aus ökonomischer Sicht nicht als Treibstoffe bzw. nicht als Edukte für die Synthese von Treibstoffen eingesetzt werden können.

Das bei dem erfindungsgemäßen Verfahren entstehende Gemisch verschiedener Kohlenwasserstoffe bietet die Möglichkeit der direkten Beimischung zu fossilem Kerosin, ohne eine Änderung der physikalischen Eigenschaften, insbesondere der Gefrierpunkte, und ohne eine vorherige Isomerisierung, wie sie beispielsweise bei der Verwendung von hydrierten Pflanzenölen durchgeführt werden muss, vornehmen zu müssen.

Ein weiterer Vorteil des erfindungsgemäßen Verfahrens gegenüber den gebräuchlichen homogen-katalysierten Guerbet-Alkoholsynthesen ist weiterhin, dass einerseits keine wässrigen salzhaltigen Abfälle durch die Neutralisation von hochkonzentrierten basischen Katalysatoren, wie etwa Kalilauge, entstehen und dass andererseits keine Korrosionsgefahr bei der Verwendung von üblichem Reaktoren, die aus rostfreiem Industriestahl (z. B. Stahlwerkstoff 1.4571 bzw. AISI 316 Ti) gefertigt sind, besteht.

Bislang existiert kein vergleichbares heterogen-katalytisches Verfahren zur Herstellung von Kerosin und Guerbet-Alkoholen bzw. allgemein von höheren Alkoholen, Aldehyden, Ketonen, Aromaten und/oder Alkanen, welches derart einfach durchzuführen und flexibel bezüglich der Rohstoffauswahl ist und sowohl zur Produktion von wertvollen Chemieprodukten als auch zur gezielten Herstellung von Flugbenzin, insbesondere Kerosin, dient.

Geeignete Katalysatoren zur Durchführung des erfindungsgemäßen Verfahrens bestehen insbesondere aus basischer bzw. mit einer basischen Ausrüstung versehenen Formaktivkohle, welche insbesondere aus einem kohlenstoffhaltigen Material bestehen, das mit einem Bindemittel und Dotierungsreagenzien vermischt und thermisch mit einem Aktivierungsgas behandelt wurde, wie nachfolgend noch ausgeführt wird. Die katalytische Aktivität beruht insbesondere auf einer Kombination der Eigenschaften des kohlenstoffhaltigen Trägers und der Dotierungsreagenzien.

Teure Edelmetalldotierungen sind für das beschriebene Verfahren der Kohlenstoffkettenverlängerung aus Alkoholen nicht notwendig, können aber optional auch als zusätzliche Dotierung eingesetzt werden, wenn dies aus Gründen der Selektivität, der Reaktionsführung oder der Verfahrenstechnik gewünscht sein sollte.

Darüber hinaus ist der zusätzliche Einsatz von Wasserstoff optional. In jedem Fall wird durch das erfindungsgemäße Verfahren - im Vergleich zur Hydrierung von Fetten und Ölen - Wasserstoff eingespart.

Die eingesetzten Katalysatoren besitzen eine hohe Lebensdauer und Wassertoleranz. Ebenfalls kann im Rahmen der vorliegenden Erfindung auf die Zugabe eines Inertgases, insbesondere Stickstoff, verzichtet werden.

Die mit dem erfindungsgemäßen Verfahren erhaltenen Produkte bzw. Produktgemische können dabei ohne weitere Aufbereitung, insbesondere ohne weitere Umsetzung, als Kraftstoff oder Kraftstoffadditiv für handelsübliche Kraftfahrzeugmotoren eingesetzt werden.

Durch die gezielte Auswahl der Edukte bzw. Eduktgemische kann die Produktverteilung bzw. das erhaltene Produktgemisch auf nahezu jedes beliebige Anforderungsprofil zugeschnitten werden.

Des Weiteren ist das erfindungsgemäße Verfahren auch in der eigentlichen Verfahrensführung sowie der Wahl der einsetzbaren Edukte äußerst flexibel: So kann das erfindungsgemäße Verfahren beispielsweise drucklos, d. h. bei Atmosphärendruck, oder unter Druck sowie in der Gasphase oder in der Flüssigphase durchgeführt werden, wobei jeweils sehr gute Umsätze und Selektivitäten erzielt werden.

Gleichfalls können als Edukte nicht nur Reinstoffe bzw. Gemische von Reinstoffen eingesetzt werden, sondern auch Fermentationsprodukte oder Fermentationsnebenprodukte, wie sie beispielsweise bei der Herstellung von Bioethanol entstehen, oder auch industrielle Abfallprodukte. Durch das erfindungsgemäße Verfahren können auch diese Neben- bzw. Abfallprodukte wieder der Wertschöpfung zugeführt und zu wertvollen Produkten bzw. Rohstoffen umgesetzt werden.

Wie oben bereits ausgeführt, wird im Rahmen des erfindungsgemäßen Verfahrens als ein Edukt mindestens eine Oxo- und/oder Hydroxylfunktion enthaltende organische Verbindung (A) eingesetzt, welche mindestens 3 Kohlenstoffatome aufweist und ausgewählt ist aus der Gruppe von primären und/oder sekundären Alkoholen, Aldehyden und Ketonen (einschließlich Hydroxyketonen) sowie deren Mischungen.

Dabei kann es im Rahmen der vorliegenden Erfindung vorgesehen sein, dass die mindestens eine Oxo- und/oder Hydroxylfunktion enthaltende organische Verbindung (A) ausgewählt ist aus C₃-C₂₅-Verbindungen, insbesondere C₃-C₂₀-Verbindungen, vorzugsweise C₃-C₁₅-Verbindungen, bevorzugt C₃-C₁₀-Verbindungen, besonders bevorzugt C₃-C₈-Verbindungen, sowie Gemischen verschiedener Verbindungen mit der vorgenannten Kohlenstoffanzahl.

Gleichfalls kann es vorgesehen sein, dass die mindestens eine Oxo- und/oder Hydroxylfunktion enthaltende organische Verbindung (A) ausgewählt ist aus linearen oder verzweigten Verbindungen. Im Rahmen der erfindungsgemäßen Verfahrensführung kann somit ein breites Spektrum an möglichen Verbindungen (A) eingesetzt werden, d. h. das erfindungsgemäße Verfahren ist äußerst flexibel und unkritisch durchführbar in Bezug auf die Wahl der mindestens eine Oxo- und/oder Hydroxylfunktion enthaltenden organischen Verbindung (A).

Unter dem Begriff "primärer und/oder sekundärer Alkohol" sind im Rahmen der vorliegenden Erfindung nicht nur primäre und sekundäre Alkohole im eigentlichen Sinne zu verstehen, sondern auch Diole und Polyole, welche mindestens eine primäre und/oder sekundäre Alkoholgruppe aufweisen. Insbesondere umfasst der Begriff "primärer und/oder sekundärer Alkohol" im Rahmen der vorliegenden Erfindung auch vicinale Diole, insbesondere Glykole, wie beispielsweise 1,2-Propandiol, welches im Rahmen der vorliegenden Erfindung sowohl als primärer als auch als sekundärer Alkohol anzusehen ist.

Wie vorstehend ausgeführt, wird im Rahmen des erfindungsgemäßen Verfahrens als zweites Edukt eine sogenannte CH-acide Verbindung (B) eingesetzt. Im Rahmen der vorliegenden Erfindung soll unter dem Begriff "CH-acide" insbesondere verstanden werden, dass bei einer C-H-Bindung, insbesondere in Gegenwart geeigneter Basen, ein Proton abgespalten wird und eine negative Ladung an dem Kohlenstoffatom verbleibt. Als CH-Acidität bezeichnet man mit anderen Worten insbesondere die Neigung einer organisch-chemischen Verbindung, an einem Kohlenstoffatom gebundene Wasserstoffatome als Protonen abzugeben und damit formal als Säure ("Brönstedt-Säure" bzw. Protonendonor) zu agieren bzw. zu fungieren.

Auch die im Rahmen der vorliegenden Erfindung als weiteres Edukt eingesetzte CH-acide Verbindung (B) kann aus einer Vielzahl von möglichen Verbindungen ausgewählt werden, insbesondere auch aus Gemischen verschiedener Verbindungen.

Erfindungsgemäß weist die CH-acide Verbindung (B) mindestens 3 Kohlenstoffatome auf. Im Allgemeinen ist die CH-acide Verbindung (B) ausgewählt aus C₃-C₂₅-Verbindungen, insbesondere C₃-C₂₀-Verbindungen, vorzugsweise C₃-C₁₅-Verbindungen, bevorzugt C₃-C₁₀-Verbindungen, besonders bevorzugt C₃-C₈-Verbindungen, sowie Gemischen verschiedener Verbindungen mit der vorgenannten Kohlenstoffanzahl.

Im Rahmen der vorliegenden Erfindung ist es vorgesehen, dass die CH-acide Verbindung (B) ausgewählt ist aus der Gruppe von primären und/oder sekundären Alkoholen, Carbonsäuren, Carbonsäureanhydriden, Carbonsäureestern, Aldehyden, Ketonen (einschließlich Hydroxyketonen), Nitrilen, Nitroverbindungen, organischen Nitraten und deren Mischungen.

Besonders gute Ergebnisse werden im Rahmen der vorliegenden Erfindung erhalten, wenn die CH-acide Verbindung (B) ausgewählt ist aus der Gruppe von primären und/oder sekundären Alkoholen, Carbonsäureanhydriden, Carbonsäureestern, Aldehyden, Ketonen (einschließlich Hydroxyketonen), Nitrilen, Nitroverbindungen, organischen Nitraten und deren Mischungen, vorzugsweise aus der Gruppe von primären und/oder sekundären Alkoholen, Aldehyden, Ketonen, Nitroverbindungen und deren Mischungen.

Insbesondere werden im Rahmen der vorliegenden Erfindung besonders gute Ergebnisse erhalten, wenn bevorzugt primäre und/oder sekundäre Alkohole, Aldehyde und Ketone, jeweils linear oder verzweigt, mit einer Kettenlänge von 4 bis 8 Kohlenstoffatomen sowie deren Mischungen als CH-acide Verbindung (B) eingesetzt werden.

Alternativ kann die CH-acide Verbindung (B) ausgewählt sein aus Nitroalkanen, insbesondere Nitromethan und/oder Nitroethan.

Durch den Einsatz von Nitroalkanen, insbesondere durch die Umsetzung von Nitromethan und/oder Nitroethan mit Alkoholen, Aldehyden und Ketonen, sind insbesondere C₆-C₁₀-Nitroalkane zugänglich, welche nach dem Stand der Technik insbesondere handelsüblichen Dieselkraftstoffen als Cetanzahlverbesserer bzw. als sogenannter Dieselbooster zugesetzt sind.

Weiterhin wird es im Rahmen der vorliegenden Erfindung bevorzugt, dass die CH-acide Verbindung (B) mindestens ein acides Wasserstoffatom an einem Kohlenstoffatom in vicinaler und/oder alpha-Position zu mindestens einem mindestens eine elektronenziehende Gruppe aufweisenden Kohlenstoffatom und/oder mindestens ein acides Wasserstoffatom an einem Kohlenstoffatom in geminaler Position zu einer elektronenziehende Gruppe aufweist.

Der Begriff "vicinal" bzw. "geminal" definiert dabei die Position des mindestens ein acides Wasserstoffatom tragenden Kohlenstoffatoms und damit die Position des aciden Wasserstoffatoms zu der elektronenziehenden Gruppe.

Der Begriff "vicinale Position" umschreibt dabei im Rahmen der vorliegenden Erfindung insbesondere, dass das die elektronenziehende Gruppe tragende Kohlenstoffatom dem den aciden Wasserstoff tragenden Kohlenstoffatom unmittelbar benachbart ist bzw. unmittelbar hieran gebunden ist.

Unter dem Begriff "geminale Position" dagegen ist im Rahmen der vorliegenden Erfindung zu verstehen, dass die elektronenziehende Gruppe an dasjenige Kohlenstoffatom gebunden ist, welches auch den aciden Wasserstoff trägt.

Unter dem Begriff "alpha-Position" soll im Rahmen der vorliegenden Erfindung ein zu einem ersten Kohlenstoffatom unmittelbar benachbartes bzw. hieran unmittelbar gebundenes zweites Kohlenstoffatom verstanden werden. Befindet sich beispielsweise das einen aciden Wasserstoff tragende Kohlenstoffatom in alpha-Position zu einem eine Oxogruppe tragenden Kohlenstoffatom, so sind acider Wasserstoff einerseits und Oxogruppe (d.h. elektronenziehende Gruppe) andererseits vicinal zueinander positioniert bzw. angeordnet.

Für den Fall, dass das acide Wasserstoffatom an einem Kohlenstoffatom in vicinaler und/oder alpha-Position zu mindestens einem mindestens eine elektronenziehende Gruppe aufweisenden Kohlenstoffatom befindlich ist, kann es vorgesehen sein, dass die elektronenziehende Gruppe ausgewählt ist aus Oxogruppen, Hydroxylgruppen, Nitrogruppen und/oder Nitratguppen. Alternativ kann es für den Fall, dass das acide Wasserstoffatom in geminaler Position zu einer elektronenziehende Gruppe befindlich ist, vorgesehen sein, dass die elektronenziehende Gruppe ausgewählt ist aus Nitrilgruppen, Isonitrilgruppen, Carbonylgruppen, Carboxylgruppen, Carbonsäureestergruppen und/oder Alkoholgruppen.

Mit anderen Worten ausgedrückt, kann es im Rahmen der vorliegenden Erfindung somit vorgesehen sein, dass die CH-acide Verbindung mindestens ein mindestens eine elektronenziehende Gruppe aufweisendes Kohlenstoffatom und/oder mindestens eine elektronenziehende Gruppe einerseits sowie mindestens ein acides Wasserstoffatom andererseits aufweist, wobei das acide Wasserstoffatom an einem Kohlenstoffatom in vicinaler und/oder alpha-Position zu dem mindestens eine elektronenziehende Gruppe aufweisenden Kohlenstoffatom oder aber in geminaler Position zu der elektronenziehenden Gruppe befindlich sein kann. Für den Fall, dass das acide Wasserstoffatom an einem Kohlenstoffatom in vicinaler und/oder alpha-Position zu mindestens einem mindestens eine elektronenziehende Gruppe aufweisenden Kohlenstoffatom befindlich ist, kann es dabei vorgesehen sein, dass die elektronenziehende Gruppe ausgewählt ist aus Oxogruppen, Hydroxylgruppen, Nitrogruppen und/oder Nitratgruppen. Für den Fall dagegen, dass das acide Wasserstoffatom in geminaler Position zu einer elektronenziehenden Gruppe befindlich werden, kann die elektronenziehende Gruppe ausgewählt ist aus Nitrilgruppen, Isonitrilgruppen, Carbonylgruppen, Carboxylgruppen, Carbonsäureestergruppen und/oder Alkoholgruppen.

Besonders gute Ergebnisse werden im Rahmen der vorliegenden Erfindung erhalten, wenn die CH-acide Verbindung (B) ausgewählt ist aus primären und/oder sekundären Alkoholen der allgemeinen Formel (I) 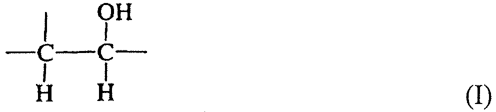 und/oder
Nitroverbindungen und/oder organischen Nitraten der allgemeinen Formel (II) 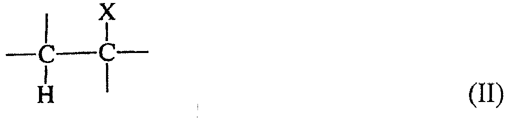 mit X = NO₂, ONO₂
und/oder
Carbonylverbindungen der allgemeinen Formel (III) 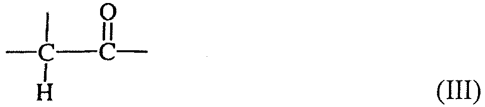 und/oder
Nitrilverbindungen der allgemeinen Formel (IV)

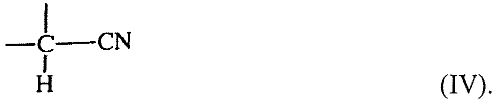

Besonders gute Ergebnisse werden dabei erhalten, wenn die primären und/oder sekundären Alkohole der allgemeinen Formel (I') entsprechen 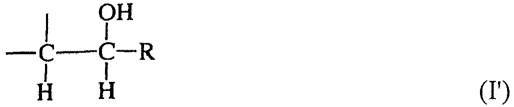 mit R = H, Organyl, insbesondere Alkyl, vorzugsweise C₁-C₄-Alkyl,
und/oder
wenn die Carbonylverbindungen der allgemeinen Formel (III') entsprechen 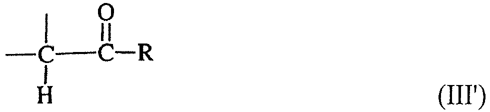 mit R = H; Organyl, insbesondere C₁-C₁₅-Alkyl, vorzugsweise C₁-C₁₀-Alkyl, bevorzugt C₁-C₆-Alkyl, besonders bevorzugt C₁-C₄-Alkyl; OH; OR¹ mit R¹ = Alkyl, insbesondere C₁-C₁₅-Alkyl, vorzugsweise C₁-C₁₀-Alkyl, bevorzugt C₁-C₆-Alkyl, besonders bevorzugt C₁- und/oder C₂-Alkyl, Aryl, Arylalkyl, Alkylaryl.

In diesem Zusammenhang können in den genannten Verbindungsklassen auch cyclische Verbindungen eingesetzt werden, insbesondere solche, bei denen die elektronenziehende Gruppe unmittelbar an das Ringsystem gebunden ist oder Teil des Ringsystems ist.

Obwohl die vorliegende Erfindung die Kondensation von organischen Verbindungen mit mehr als drei Kohlenstoffatomen betrifft, kann es vorgesehen sein, dass den Edukten zusätzlich niedere Alkohole, insbesondere Methanol und/oder Ethanol, vorzugsweise Ethanol, zugesetzt sind, welche dann an der Reaktion teilnehmen. Die niederen Alkohole, insbesondere Ethanol, können den Edukten entweder zugemischt werden oder aber bereits von Anfang an in diesen enthalten sein. So können beispielsweise Fermentationsprodukte von Glucose oder Cellulose, wie beispielsweise Aceton, Butanol und Ethanol, die sogenannten ABE-Produkte, zugesetzt werden. Die Anwesenheit von niederen Alkoholen erhöht zudem den Umsatz der Kondensations- bzw. Kopplungsreaktion und verbessert gleichzeitig die Gesamtproduktselektivität.

Darüber hinaus hat es sich im Rahmen der vorliegenden Erfindung als vorteilhaft erwiesen, wenn den Edukten Wasser zugesetzt wird. Durch die Anwesenheit von Wasser während der Reaktion lässt sich insbesondere die Produktselektivität gezielt steuern, insbesondere deutlich erhöhen.

Für das erfindungsgemäße Verfahren bzw. die erfindungsgemäße Verfahrensführung ist die Anwesenheit von Wasser einerseits unkritisch, d. h. das Verfahren wird nicht negativ beeinflusst; andererseits können die Produktselektivitäten sogar noch verbessert werden. Dies hat beispielsweise zur Folge, dass Fermentationsprodukte oder industrielle Abfallprodukte ohne besondere Reinigungsschritte, insbesondere ohne die vollständige bzw. überwiegende Entfernung von Wasseranteilen, im erfindungsgemäßen Verfahren als Edukte eingesetzt werden können, wobei der Einsatz solcher wasserhaltiger Eduktgemische gegenüber reinen und wasserfreien Edukten erfindungsgemäß sogar bevorzugt ist. Hierdurch können Zeit und Energie eingespart werden, wodurch die ökonomischen und ökologischen Ressourcen weniger belastet werden.

Mit dem erfindungsgemäßen Verfahren können auch Methanol und Ethanol oder höhere Alkohole aus der Synthesegasherstellung, wie etwa aus dem Fischer-Tropsch-Alkohol-Prozess und alle fermentativ hergestellten Alkohole oder deren Gemische als Einsatzstoffe genutzt werden. Gerade dies macht das erfindungsgemäße Verfahren gegenüber Verfahren des Standes der Technik deutlich flexibler. Insbesondere ist es von Vorteil, dass auch durch Fermentation hergestellte Alkohole, alleine oder in Kombination mit anderen biobasierten Alkoholen, genutzt werden können und sogar als Azeotrop mit Wasser unmittelbar in dem erfindungsgemäßen Verfahren eingesetzt werden können, so dass eine aufwendige Abtrennung von Wasser vor der Durchführung des erfindungsgemäßen Verfahrens nicht notwendig ist.

Besonders vorteilhaft und nachhaltig lässt sich das erfindungsgemäße Verfahren durchführen, wenn sogenannte Fuselalkohole aus der Fermentation von Abfallströmen oder aus der Fermentation von Kohlenmonoxid, wie etwa aus Konvertergas aus der Stahlgewinnung, als Rohstoff einsetzt werden. Weiterhin ist es vorteilhaft, dass das Wasser in den aus der Fermentation gewonnen Fuselalkoholen die Selektivität der Alkoholkondensation zu Alkanen sogar fördert.

Aufgrund der guten Wassertoleranz des erfindungsgemäßen Verfahrens ist es auch möglich, die bei einer Fermentation entstehenden Gemische, wie z. B. Aceton/Butanol/Ethanol (ABE-Synthese), direkt einzusetzen, insbesondere nach einer vorgeschalteten Verringerung des Wassergehaltes.

Ebenfalls vorteilhaft ist es, dass mit dem erfindungsgemäßen Verfahren C₄-C₈-Alkohole umgesetzt werden können, die zuvor aus der Kondensation von Ethanol alleine oder im Gemisch mit Methanol hergestellt wurden und zusätzlich auch Aldehyde und Wasser enthalten können.

Gemäß einer besonderen Ausführungsform der vorliegenden Erfindung kann es vorgesehen sein, dass die mindestens eine Oxo- und/oder Hydroxylfunktion enthaltende organische Verbindung (A) einerseits und die CH-acide Verbindung (B) andererseits identisch sind. Im Rahmen der vorliegenden Erfindung ist es somit möglich, auch sogenannte Autokondensationsreaktionen, insbesondere von Alkoholen, Aldehyden oder Ketonen, durchzuführen.

Weiterhin können durch den Einsatz von sekundären n-Alkoholen oder deren Oxidationsprodukten, wie etwa 2-Propanol bzw. Aceton, zwei Moleküle eines Alkohols an die jeweiligen, der funktionellen Gruppe des sekundären Alkohols respektive des Ketons benachbarten Kohlenstoffatome gekoppelt werden, was die Flexibilität bezüglich der möglichen Reaktionsprodukte deutlich erhöht. Auch der Einsatz von verzweigten Alkoholen und deren Oxidationsprodukten ist dabei möglich.

Insbesondere ist es im Rahmen der vorliegenden Erfindung auch möglich, sekundäre Alkohole und/oder Ketone, wie beispielsweise 2-Propanol oder Aceton, mit verzweigten längerkettigen primären und/oder sekundären Alkohlen, insbesondere Guerbet-Alkoholen, wie z. B. 2-Ethylbutanol, 2-Ethyloctanol etc., zu kondensieren. Auf diese Weise können stärker verzweigte Verfahrensprodukte, insbesondere Alkohole und Alkane, erhalten werden, deren Schmelzpunkte im Vergleich zu den unverzweigten Produkten üblicherweise um 10 °C oder mehr erniedrigt sind. Derartige Produkte eignen sich in besonderem Maße zur Verwendung als Flugbenzin, insbesondere Kerosin, oder dessen Vorläufersubstanzen bzw. Präkursoren.

Die bei der erfindungsgemäßen Verfahrensführung ablaufenden Kopplungs- bzw. Kondensationsreaktionen sollen im Folgenden exemplarisch anhand der nachfolgenden Reaktionsgleichungen (1) und (2) erläutert werden.

Reaktionsgleichung (1) beschreibt die erfindungsgemäße Umsetzung von n-Hexanol mit Aceton einerseits ("Synthese a)") sowie mit 2-Propanol andererseits ("Synthese b)"). In beiden Varianten der Verfahrensführung werden identische Verbindungen erhalten, wobei das Keton bzw. der sekundäre Alkohol als CH-acide Verbindung fungieren. Aus der Kopplungsreaktion von n-Hexanol mit Aceton oder 2-Propanol wird das zweifache Kondensationsprodukt als Hauptprodukt erhalten, während das einfache Kondensationsprodukt sowie ein Oxidationsprodukt des n-Hexanols, nämlich Hexanal, als vorrangige Nebenprodukte erhalten werden.

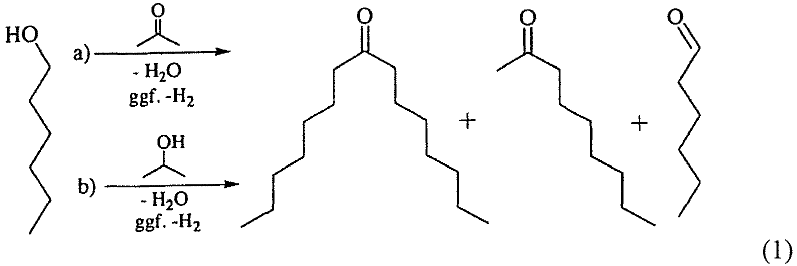

Reaktionsgleichung (2) verdeutlicht hingegen die Herstellung von sogenannten Guerbet-Alkoholen sowie von verzweigten Alkanen am Beispiel der Autokondensation von n-Hexanol.

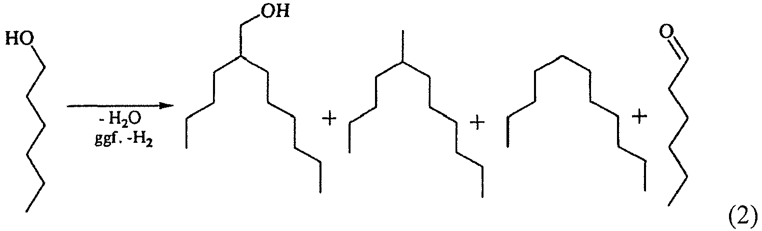

Sowohl Reaktionsgleichung (1) als auch Reaktionsgleichung (2) geben den jeweiligen Reaktionsverlauf nur vereinfacht wieder, wobei nicht alle entstehenden Produkte in den Reaktionsgleichungen aufgeführt sind.

Doch nicht nur die Wahl der Edukte, sondern auch die weiteren Prozessparameter besitzen einen großen Einfluss auf die Effizienz und Selektivität des erfindungsgemäßen Verfahrens sowie auf die Ausbeute und die Produktverteilung:
Im Allgemeinen wird das Verfahren im Rahmen der erfindungsgemäßen Verfahrensführung in der Gasphase und/oder in der Flüssigphase und/oder im überkritischen Bereich durchgeführt, wobei eine Verfahrensführung in der Gasphase bevorzugt wird.

Weiterhin kann es vorgesehen sein, dass das Verfahren oberhalb der Siedetemperatur der Edukte und/oder der Produkte, vorzugsweise oberhalb der Siedetemperaturen der Edukte und Produkte, durchgeführt wird.

Besonders gute Umsätze, Ausbeuten und Selektivitäten werden erhalten, wenn das Verfahren bei Temperaturen im Bereich von 150 °C bis 600 °C, insbesondere 250 bis 450 °C, vorzugsweise 300 bis 400 °C, durchgeführt wird.

Weiterhin kann im Rahmen der vorliegenden Erfindung das Verfahren grundsätzlich bei reduziertem Druck, bei Atmosphärendruck oder bei erhöhtem Druck durchgeführt werden. In diesem Zusammenhang hat es sich jedoch als besonders vorteilhaft erwiesen, wenn das Verfahren bei Atmosphärendruck oder bei erhöhtem Druck durchgeführt wird; sofern das Verfahren bei Atmosphärendruck oder erhöhtem Druck durchgeführt wird, liegt der Absolutdruck im Allgemeinen im Bereich von Atmosphärendruck bis 150 bar, insbesondere im Bereich von Atmosphärendruck bis 80 bar, vorzugsweise im Bereich von Atmosphärendruck bis 25 bar.

Wie oben bereits dargelegt, bewirkt eine Steigerung des Drucks und/oder der Temperatur im Allgemeinen eine Erhöhung der Umsätze, wobei aber die Erhöhung des Umsatzes ab einem gewissen Punkt auf Kosten der Selektivität erreicht wird, so dass für jeden Fall die optimale Abstimmung der einzelnen Prozessparameter aufeinander bestimmt werden muss.

Auch die Reaktionsdauern bzw. die Kontaktzeiten besitzen einen großen Einfluss auf die Umsätze und Ausbeuten einerseits und die Selektivität der Produktbildung andererseits. Im Allgemeinen wird das Verfahren mit Reaktionsdauern und/oder Kontaktzeiten im Bereich von 0,01 min bis 12 Stunden, insbesondere 0,1 min bis 10 Stunden, vorzugsweise 1 min bis 5 Stunden durchgeführt.

Falls das Verfahren in der Gasphase und/oder im überkritischen Bereich durchgeführt wird, so hat es sich bewährt, wenn das Verfahren mit Reaktionsdauern und/oder Kontaktzeiten im Bereich von 0,001 bis 120 Sekunden, insbesondere 0,001 bis 60 Sekunden, vorzugsweise 0,05 bis 30 Sekunden, durchgeführt wird.

Wird das erfindungsgemäße Verfahren hingegen in der Flüssigphase durchgeführt, so werden gute Ergebnisse erhalten, wenn das Verfahren mit Reaktionsdauern und/oder Kontaktzeiten im Bereich von 0,001 min bis 12 Stunden, insbesondere 0,1 min bis 10 Stunden, vorzugsweise 1 min bis 5 Stunden, durchgeführt wird.

Im Rahmen der vorliegenden Erfindung kann es - wie zuvor angeführt - darüber hinaus vorgesehen sein, dass das Verfahren in Gegenwart von Wasser, vorzugsweise in Form von Wasserdampf, und/oder in Gegenwart von Wasserstoff durchgeführt wird. Das Wasser kann dabei einerseits während der Kondensations- bzw. Kopplungsreaktion entstehen und/oder andererseits den Edukten oder im Laufe der Verfahrensführung zugesetzt werden, wobei die Gegenwart einer gewissen Menge an Wasser die Produktselektivität der Kondensationsreaktion bzw. Kopplungsreaktion deutlich erhöht.

Gleichfalls ist es auch möglich, die Reaktion in Gegenwart von Wasserstoff durchzuführen, wobei im Laufe der Reaktion im Allgemeinen gleichfalls Wasserstoff gebildet wird. Wird jedoch zusätzlich Wasserstoff zugesetzt, so kann durch Hydrierungsreaktionen der Anteil an Aldehyden und Ketonen im erhaltenen Produktgemisch reduziert bzw. minimiert werden, wobei gleichzeitig der Anteil an Alkoholen und Alkanen erhöht bzw. maximiert wird.

Im Rahmen des erfindungsgemäßen Verfahrens können den Edukten bzw. Ausgangsstoffen darüber hinaus auch Inertgase, insbesondere Stickstoff und/oder Argon, vorzugsweise Stickstoff, zugesetzt werden, falls dies gewünscht oder erforderlich ist. Die Zugabe der Inertgase erfolgt insbesondere zu Verdünnungszwecken, was oftmals mit einer verbesserten Produktselektivität einhergeht.

Alternativ oder ergänzend kann das erfindungsgemäße Verfahren auch in Gegenwart weiterer Gase durchgeführt werden. So können den Edukten oder während der Verfahrensdurchführung beispielsweise kurzkettige Alkane, insbesondere lineare C₁-C₆-Alkane, zugesetzt werden, welche eine Verbesserung der Wärmeübertragung und der Selektivität bewirken.

Gleichfalls ist es möglich, Kohlenstoffmonoxid und/oder Kohlenstoffdioxid den Edukten oder während der Durchführung des Verfahrens zuzusetzen; diese Stoffe können als weitere mögliche Reaktionspartner während der Umsetzung dienen.

Die Anwesenheit von Gasen ermöglicht insbesondere eine weniger aufwendige und effizientere Trennung des Produktgemisches, falls dies gewünscht ist.

Im Allgemeinen wird es im Rahmen der vorliegenden Erfindung jedoch bevorzugt, wenn lediglich nur schwach verdünnte, vorzugsweise aber unverdünnte, Edukte bzw. Eduktgemische eingesetzt werden und auch während der Verfahrensführung keine Gase, mit Ausnahme von Wasserstoff, zugesetzt werden, da auf diese Weise die höchsten Raum-Zeit-Ausbeuten und die größten Raumgeschwindigkeiten erzielt werden können.

Das Verfahren nach der Erfindung kann grundsätzlich diskontinuierlich, d. h. chargenweise bzw. im Batchbetrieb, oder aber kontinuierlich betrieben werden. Bevorzugt, insbesondere bei technischer oder industrieller Anwendung, ist eine kontinuierliche Verfahrensführung, welche hohe Raum-Zeit-Ausbeuten bzw. Raumgeschwindigkeiten und Umsätze ermöglicht und folglich besonders wirtschaftlich durchgeführt werden kann.

Im Allgemeinen wird das erfindungsgemäße Verfahren mit einer Raum-Zeit-Ausbeute, angegeben als Menge aller gebildeter Produkte pro Katalysatorvolumen und pro Zeiteinheit, im Bereich von 10 bis 3.000 g/(Liter • h), insbesondere 25 bis 2.500 g/(Liter • h), vorzugsweise 30 bis 2.000 g/(Liter • h), besonders bevorzugt 50 bis 1.500 g/(Liter • h), durchgeführt.

Eine andere Maßzahl für die hohe Effizienz und Leistungsfähigkeit des erfindungsgemäßen Verfahrens sind die erreichten Raumgeschwindigkeiten. Das erfindungsgemäße Verfahren wird im Allgemeinen mit einer Raumgeschwindigkeit, angegeben als Stoffmenge aller gebildeter Produkte pro Katalysatormasse und pro Zeiteinheit, im Bereich von 0,1 bis 100 mol/(kg • h), insbesondere 0,5 bis 25 mol/(kg • h), vorzugsweise 1,0 bis 20 mol/(kg • h), besonders bevorzugt 1,25 bis 18 mol/(kg • h), ganz besonders bevorzugt 1,5 bis 15 mol/(kg • h), durchgeführt.

Weiterhin kann es vorgesehen sein, dass das Verfahren mit einem stoffmengenbezogenen Umsatz, bezogen auf die eingesetzten Edukte im Bereich von 15 bis 100 %, insbesondere 20 bis 90 %, vorzugsweise 30 bis 80 %, besonders bevorzugt 40 bis 75 %, durchgeführt wird.

Die oben angegebenen Raum-Zeit-Ausbeuten und Raumgeschwindigkeiten sowie die stoffmengenbezogenen Umsätze beschreiben Bereiche, in welchen das erfindungsgemäße Verfahren besonders wirtschaftlich und nach verfahrensökonomischen Gesichtspunkten günstig durchgeführt werden kann, wobei eine hohe Selektivität in den erhaltenen Produktgemischen erzielt wird.

Wie oben bereits ausgeführt, kommt im Rahmen des erfindungsgemäßen Verfahrens ein Katalysator auf Basis eines metallbeladenen Aktivkohlesubstrats zum Einsatz.

Die im Rahmen der vorliegenden Erfindung eingesetzte Aktivkohle enthält vorzugsweise nicht nur Kohlenstoff, sondern auch kleine Mengen an Sauerstoff, Stickstoff, Schwefel und Wasserstoff, welche chemisch in Form verschiedener funktioneller Gruppen, wie etwa Carbonyl-, Carboxyl-, Phenol- und Ethergruppen sowie Lactonen und Chinonen gebunden sind. Diese Oberflächenoxide können aus den Rohstoffen resultieren, oder aber sie können durch den Aktivierungsprozess, durch den Einfluss von chemischen Aktivatoren sowie durch den Einfluss von Sauerstoff oder Wasserdampf entstehen. Die chemischen Eigenschaften der Oberfläche spielen eine signifikante Rolle für die Adsorption und die Katalyse.

Die Ausgangsmaterialien für Aktivkohle, welche zur Herstellung von erfindungsgemäß einsetzbaren Katalysatoren geeignet sind, besitzen im Allgemeinen mineralische Komponenten, die während des Aktivierungsprozesses aufkonzentriert werden können. Weiterhin ist es auch möglich, dass anorganische Chemikalien für die Aktivierung der Aktivkohle nicht vollständig entfernt werden oder ganz auf der Aktivkohle verbleiben.

Der Aschegehalt von Aktivkohlen wird maßgeblich durch die mineralischen Komponenten bestimmt. Die Hauptbestandteile dieser Asche sind Alkali- und Erdalkalimetalle, zumeist in Form von Carbonaten und Phosphaten, gegebenenfalls zusammen mit Kieselsäure sowie Eisen- und Aluminiumoxiden. Der Aschegehalt von Aktivkohlen kann durch Waschen mit Wasser oder Säure reduziert werden. Kommerzielle Produkte weisen daher Aschegehalte von unter einem bis zu zwanzig Prozent auf.

Aktivkohle wirkt zugleich als Katalysator und als Katalysatorträger: Die katalytische Aktivität der Aktivkohle als solcher beruht im Wesentlichen auf der Struktur des Kohlenskeletts, die aus einer Mischung von amorphem und graphitähnlichem Kohlenstoff besteht; am Rand von Schichten gibt es viele chemisch ungesättigte Ecken und Kanten, die als so genannte Gitterlücken fungieren, und auf der internen Aktivkohleoberfläche der im Rahmen des erfindungsgemäßen Verfahrens eingesetzten Aktivkohle liegen bevorzugt die bereits genannten Oberflächenoxide, welche an Redoxreaktionen teilnehmen können und mitunter den Grund für die chemische Aktivität von Aktivkohlen darstellen. Zudem fungieren die erfindungsgemäß eingesetzten Aktivkohlen als Träger für die Metalldotierung.

Der zur Durchführung des erfindungsgemäßen Verfahrens eingesetzte Katalysator und/oder das eingesetzte Aktivkohlesubstrat sind im Allgemeinen basisch ausgerüstet und/oder ausgebildet.

Insbesondere kann es vorgesehen sein, dass der Katalysator und/oder das Aktivkohlesubstrat mindestens eine basische funktionelle Gruppe und/oder mindestens eine basische chemische Verbindung aufweist.

Dabei hat es sich insbesondere als besonders vorteilhaft erwiesen, wenn die basische Ausrüstung durch (i) Hydroxide; (ii) Oxide; (iii) Salze anorganischer Säuren, insbesondere Phosphate, Sulfate, Carbonate, und Nitrate; (iv) Salze organischer Säuren, insbesondere Lactate, Phthalate, Formiate und Acetate; und/oder (v) Alkoholate bereitgestellt wird.

Gemäß einer bevorzugten Ausführungsform der vorliegenden Erfindung wird die basische Ausrüstung durch Carbonate und/oder Phosphate, besonders bevorzugt durch Carbonate und Phosphate, bereitgestellt.

Die basische Ausrüstung kann dabei bei der Herstellung des Katalysators oder aber nachträglich, insbesondere mittels Imprägnierung, erfolgen. Besonders gute Ergebnisse werden im Rahmen der vorliegenden Erfindung jedoch erhalten, wenn die basische Ausrüstung bei der Herstellung des Katalysators erfolgt.

Unter einer basischen Ausrüstung ist im Rahmen der vorliegenden Erfindung zu verstehen, dass der Katalysator bzw. das Aktivkohlesubstrat basische Gruppen und/oder Verbindungen oder aber basisch reagierende Gruppen und Verbindungen aufweist. Entscheidend ist, dass der basische Charakter dieser Gruppen bzw. Verbindungen im fertig hergestellten Katalysator unter Reaktionsbedingungen erhalten bleiben. Dabei ist es durchaus möglich, dass die ursprünglich eingesetzten Verbindungen bei der Katalysatorherstellung oder aber bei der Katalysereaktion umgewandelt werden; in diesem Fall müssen die Umwandlungsprodukte basischen Charakter besitzen. So können beispielsweise Carbonate bei einer Aktivierung des Aktivkohlesubstrats zu Oxiden reagieren, gleichfalls ist es jedoch auch möglich, dass die Carbonate mit dem Kohlenstoffgerüst des Aktivkohlesubstrats, beispielsweise unter Bildung von Phenolaten, Oxiden, Anhydriden, Hydroxiden etc., reagieren.

Darüber hinaus sollte der erfindungsgemäß eingesetzte Katalysator eine große spezifische Oberfläche aufweisen. Der im Rahmen des erfindungsgemäßen Verfahrens eingesetzte Katalysator und/oder das Aktivkohlesubstrat weist im Allgemeinen eine spezifische Oberfläche (BET) im Bereich von 450 bis 3.000 m²/g, insbesondere 500 bis 2.500 m²/g, vorzugsweise 600 bis 2.250 m²/g, besonders bevorzugt 900 bis 1.700 m²/g, ganz besonders bevorzugt 950 bis 1.500 m²/g, noch mehr bevorzugt 1.000 bis 1.350 m²/g, auf.

Darüber hinaus sollte der erfindungsgemäß eingesetzte Katalysator ein großes Mikroporenvolumen besitzen. Insbesondere kann es vorgesehen sein, dass der Katalysator und/oder das Aktivkohlesubstrat ein Mikroporenvolumen, insbesondere ein Mikroporenvolumen nach Gurvich, im Bereich von 0,1 bis 3,0 ml/g, insbesondere 0,2 bis 2,5 ml/g, vorzugsweise 0,25 bis 1 ml/g, besonders bevorzugt 0,3 bis 0,7 ml/g, aufweist.

Weiterhin hat es sich im Rahmen der vorliegenden Erfindung als vorteilhaft erwiesen, wenn der Katalysator und/oder das Aktivkohlesubstrat eine Standzeit von mindestens 10 Tagen, insbesondere mindestens 20 Tagen, vorzugsweise mindestens 30 Tagen, besonders bevorzugt mindestens 6 Monaten, aufweist. Lange Standzeiten des erfindungsgemäß eingesetzten Katalysators ermöglichen die kontinuierliche großtechnische Durchführung des erfindungsgemäßen Verfahrens und ermöglichen somit eine in ökonomischer Hinsicht günstige Herstellung höherer Alkohole und Aldehyde.

Gleichfalls hat es sich als vorteilhaft erwiesen, wenn der Katalysator und/oder das Aktivkohlesubstrat mindestens eine funktionelle Gruppe, vorzugsweise eine polare und/oder ionische funktionelle Gruppe, umfasst. Dabei kann es vorgesehen sein, dass die mindestens eine funktionelle Gruppe ausgewählt ist aus Carbonyl-, Carboxylat-, Hydroxyl-, Oxid-, Ether-, Ester-, Lacton-, Phenol- und/oder Chinongruppen. Die vorgenannten funktionellen Gruppen können beispielsweise durch Reaktionen des Kohlestoffgerüsts des Aktivkohlesubstrats mit einer für die basische Ausrüstung benötigten Verbindung während der Aktivierung des Aktivkohlesubstrats gebildet werden (wie zuvor beschrieben).

Im Allgemeinen ist das Metall, insbesondere die Metalldotierung, des im Rahmen des erfindungsgemäßen Verfahrens eingesetzten Katalysators ausgewählt aus der Gruppe von Alkalimetallen, Erdalkalimetallen, Metallen der Nebengruppen des Periodensystems der Elemente und der Seltenen Erden sowie deren Gemischen oder Kombinationen.

Weiterhin kann es vorgesehen sein, dass der Katalysator mindestens ein einwertiges Metall M^{I}, insbesondere mindestens ein Alkalimetall, vorzugsweise Natrium und/oder Kalium, und/oder mindestens ein zweiwertiges Metall M^{II}, insbesondere Calcium und/oder Magnesium, besonders bevorzugt mindestens ein einwertiges Metall M^{I} und mindestens ein zweiwertiges Metall M^{II}, aufweist.

Gleichfalls kann es vorgesehen sein, dass der Katalysator Phosphor, insbesondere in Form von Phosphaten, enthält.

Besonders gute Ergebnisse werden im Rahmen des erfindungsgemäßen Verfahrens erhalten, wenn die vorgenannten Verbindungen und/oder Substanzen in speziellen molaren Verhältnissen zueinander in dem erfindungsgemäß eingesetzten Katalysator vorhanden sind. In diesem Zusammenhang ist es im Rahmen der vorliegenden Erfindung bevorzugt, wenn für die folgenden molaren Verhältnisse gilt:
(i) 0,5 ≤ M^{I} / M^{II} ≤ 5, insbesondere 2 ≤ M^{I} / M^{II} ≤ 3; und/oder
(ii) 2 ≤ M^{II} / P ≤ 30, insbesondere 2 ≤ M^{II} / P ≤ 8; und/oder
(iii) 1 ≤ M^{I} / P ≤ 60, insbesondere 5 ≤ M^{I} / P ≤ 10; und/oder
(iv) 1 ≤ K / Na ≤ 20, insbesondere 10 ≤ K / Na ≤ 20; und/oder
(v) 1 ≤ Ca / Mg ≤ 10, insbesondere 4 ≤ Ca / Mg ≤ 6.

Gleichfalls werden im Rahmen des erfindungsgemäßen Verfahrens sehr gute Ergebnisse erhalten, wenn der Katalysator die folgenden Mengenanteile (Gewichtsprozente) der nachfolgend genannten Komponenten umfasst, wobei die nachfolgenden Angaben jeweils auf den Katalysator bezogen sind:
(i) M^{I}, insbesondere Natrium und/oder Kalium, bevorzugt Natrium und Kalium: 0,1 bis 20 Gew.-%, insbesondere 0,2 bis 15 Gew.-%, vorzugsweise 0,5 bis 10 Gew.-%; und/oder
(ii) M^{II}, insbesondere Calcium und/oder Magnesium, bevorzugt Calcium und Magnesium: 0,1 bis 20 Gew.-%, insbesondere 0,2 bis 10 Gew.-%, vorzugsweise 0,5 bis 5 Gew.-%; und/oder
(iii) P, insbesondere in Form von Phosphat, berechnet als Phosphor P: 0,01 bis 5 Gew.-%, insbesondere 0,02 bis 2,5 Gew.-%, vorzugsweise 0,02 bis 1 Gew.-%.

Besonders gute Umsätze, Ausbeuten und Selektivitäten können im Rahmen des erfindungsgemäßen Verfahrens erzielt werden, wenn der erfindungsgemäß eingesetzte Katalysator die zuvor genannten Metalle und Phosphor sowohl in den speziellen molaren Verhältnissen zueinander als auch in den jeweiligen absoluten Stoffmengenanteilen enthält.
Insbesondere kann es im Rahmen der vorliegenden Erfindung vorgesehen sein, dass als Katalysator eine basisch ausgerüstete und/oder eingestellte Aktivkohle, welche mit mindestens einer Alkali- und/oder Erdalkalidotierung, vorzugsweise Alkali- und Erdalkalidotierung, besonders bevorzugt Kalium-und Calcium- und/oder Magnesiumdotierung, versehen ist, eingesetzt wird. Hierbei hat es sich als besonders vorteilhaft erwiesen, wenn eine mit Phosphat und/oder Carbonat basisch eingestellte, mit Kalium und Calcium und/oder Magnesium dotierte Aktivkohle eingesetzt wird.

Gemäß einer besonderen Ausführungsform der vorliegenden Erfindung kann als Katalysator eine Formaktivkohle eingesetzt werden, wie sie in der DE 10 2004 033 561 A1 und der DE 10 2004 033 561 B4 beschrieben ist. Im Rahmen der vorliegenden Erfindung kommt als Katalysator eine Formaktivkohle zum Einsatz, welche herstellbar ist durch ein Verfahren zur Herstellung von Formaktivkohle aus einem Kohlenstoffträger, einem Bindemittel und einer katalytischen Komponente der allgemeinen Formel (I)

[M]ₘ₃[AOₙ₄]ₘ₄ (I)

wobei
- M ein Kation bezeichnet und ausgewählt ist aus der Gruppe von Alkali- oder Erdalkalimetallkationen;
- m3 und m4 stöchiometrische Koeffizienten mit ganzen Zahlen mit m3 ≥ 1 und m4 ≥ 1 bezeichnen;
- [AOₙ₄] ein sauerstoffhaltiges Anion mit dem ganzzahligen stöchiometrischen Koeffizienten n4 ≥ 1 bezeichnet;
- [AOₙ₄] vorzugsweise ausgewählt wird aus der Gruppe von Carbonaten oder Hydroxiden,
wobei das Bindemittel erhalten wird aus der Umsetzung eines wasserlöslichen kohlenhydrathaltigen Ausgangsstoffes mit einem Glucosegehalt von ≥ 50 Gew.-%, insbesondere von ≥ 60 Gew.-%, wobei der Kohlenstoffträger zunächst mit der katalytischen Komponente vermischt wird, wobei anschließend die Mischung aus katalytischer Komponente und Kohlenstoffträger mit dem Bindemittel vermischt wird, wobei die so erhaltene Mischung aus Kohlenstoffträger, katalytischer Komponente und Bindemittel zu Formlingen verpresst wird und wobei die Formlinge carbonisiert und aktiviert werden, wobei das Bindemittel aus der Umsetzung des kohlenhydrathaltigen Ausgangsstoffes mit einem Zuschlagstoff erhalten wird, wobei zum Erhalt des Bindemittels der Zuschlagstoff dem kohlenhydrathaltigen Ausgangsstoff zugegeben wird vor der Vermischung des Bindemittels mit der Mischung aus dem Kohlenstoffträger und der katalytischen Komponente, wobei der Zuschlagstoff ausgewählt ist aus der Gruppe von Phosphorsäuren und/oder deren Salzen, Schwefelsäuren und/oder deren Salzen und/oder Schwefelsäurederivaten und/oder deren Salzen.

Dieses allgemeine Herstellungsverfahren und die chemische Zusammensetzung der Verbindungen der Metalle, Übergansmetalle und Seltenen Erden in der Dotierung zielen ursprünglich auf die Adsorption von sauren Gasen ab. Zur Herstellung eines im erfindungsgemäßen Verfahren einsetzbaren, besonders wirkungsvollen Katalysators kann dieses Herstellungsverfahren noch geringfügig adaptiert werden, wobei die Adaptionen vollumfänglich in der DE 10 2004 033 561 A1 bzw. der DE 10 2004 033 561 B4 offenbart sind.

Als Dotierungsreagenz für die Alkoholsynthese werden für die Formaktivkohle für die Alkohol- bzw. Aldehydsynthese Metallsalze eingesetzt, deren Kationen ausgewählt sind aus den Metallen der 1. und 2. Hauptgruppe, den Übergangsmetallen, den Seltenen Erden und den Halbmetallen.

Vorzugsweise wird dem Kohlenstoffträger K₂CO₃ als Aktivator zugesetzt. Kaliumcarbonat reagiert mit dem Kohlenstoffträger unter anderem unter Kohlenstoffverbrauch und führt zur Bildung von sehr kleinen Mikroporen, die während der Gasaktivierung mit Wasserdampf weiter zu größeren Mikroporen und Mesoporen aufgeweitet werden und so zum gewünschten Porensystem führen. Durch Variation der Menge an K₂CO₃ in dem Kohlenstoffträger und der Aktivierungsbedingungen (Temperatur, Wasserdampfmenge, Verweilzeit etc.) lassen sich deswegen unterschiedliche Porengrößen und Porenverteilungen in der Formaktivkohle einstellen.

Die vorgesehenen Zuschlagstoffe, wie etwa K₂CO₃, müssen noch vor der Vermischung des Bindemittels mit dem Kohlenstoffträger dem kohlenhydrathaltigen Ausgangsstoff des Bindemittels zugegeben werden. Das Bindemittel zur Herstellung von Formaktivkohle ist erhältlich aus der Umsetzung eines wasserbasierten glucosehaltigen Ausgangsstoffes mit einem Zuschlagstoff, wobei der Zuschlagstoff ausgewählt ist aus der Gruppe der Phosphorsäuren und/oder deren Salzen. Bei dem wasserbasierten glucosehaltigen Ausgangsstoff handelt es sich vorzugsweise um Glucose oder Glucosederivate, wie vorzugsweise Glucosesirup, Dicksaft oder Fruchtsirup. Diese zuckerhaltigen Ausgangsstoffe zeichnen sich durch einen kleinen Ascheanteil von < 5 Gew.-%, insbesondere von < 2 Gew.-%, aus, was ebenfalls für die Eigenschaften der Formaktivkohle von Vorteil ist. Grundsätzlich können alle Kohlenhydrate, beispielsweise Monosaccharide (insbesondere Glucose, Fructose, Mannose, Galactose etc.) und/oder Disaccharide (insbesondere Saccharose, Maltose, Lactose, Cellobiose, Trehalose etc.) und/oder Tri-, Tetra-, Oligo- und Polysaccharide (insbesondere Stärke, Cellulose, Glykogen etc.) und/oder vorgelöste Stärke oder Cellulose, insbesondere in Form von wässrigen Lösungen, als Ausgangsstoffe eingesetzt werden. Auch Mischungen verschiedenster Zucker können eingesetzt werden.

Wird als Zuschlagstoff für die Umsetzung des wasserbasierten glucosehaltigen Ausgangsstoffes zu einem Bindemittel Phosphorsäure ausgewählt, ist es vorzugsweise vorgesehen und von Vorteil, dass nach dem Vermischen der Phosphorsäure mit dem wasserbasierten glucosehaltigen Ausgangsstoff das so erhältliche Bindemittel nicht neutralisiert wird. Wird dieses Bindemittel anschließend zur Herstellung von Formaktivkohle mit einem Kohlenstoffträger vermischt, kommt es zur Neutralisation saurer Gruppen des Bindemittels mit basischen Gruppen des Kohlenstoffträgers. Durch den Verzicht auf die Verfahrensstufe der Neutralisation wird der Herstellungsaufwand bei der Herstellung des Bindemittels deutlich vereinfacht. Darüber hinaus ist es auch möglich und ebenso von Vorteil, direkt ein Salz einer Phosphorsäure als Zuschlagstoff zu dem wasserbasierten glucosehaltigen Ausgangsstoff bei der Herstellung des Bindemittels einzusetzen.

Im Rahmen dieser Ausführungsform ist es bevorzugt, wenn der Zuschlagstoff der allgemeinen Formel (II)

[Mₘ₁][Hₙ₁Pₙ₂Oₙ₃]ₘ₁ (II)

entspricht,
- wobei M ein Proton (H⁺) oder ein Kation, welches ausgewählt ist aus der Gruppe von Alkali-, Erdalkali-, Ammonium-, Calcium-, Magnesium- und Eisenionen, vorzugsweise aus Alkali-, Erdalkali- und Ammoniumionen, bezeichnet, wobei H Wasserstoff und P bzw. O Phosphor bzw. Sauerstoff bezeichnen,
- wobei m1 und m2 stöchiometrische Koeffizienten bezeichnen und ganze Zahlen mit m1 ≥ 1 und m2 ≥ 1 sind;
- wobei [Hₙ₁Pₙ₂Oₙ₃] ein Anion bezeichnet mit ganzzahligen stöchiometrischen Koeffizienten n1, n2 und n3 mit n1 > 0, n2 > 1; n3 > 2.

Als Zuschlagstoff für das kohlenhydrathaltige bzw. zuckerhaltige Bindemittel ist insbesondere Phosphorsäure (H₃PO₄) geeignet.

In Gegenwart von Phosphorsäuren wird der kohlenhydrathaltige Ausgangsstoff unter Bildung von Kohlenstoff dehydratisiert. Dieser Vorgang ist am Beispiel von Glucose in der nachfolgenden Gleichung illustriert:

C₁₂H₂₂O₁₁ → 12 C+ 11 H₂O

Dabei entsteht eine Kohlenstoffmodifikation, die - im Vergleich zum zugesetzten Kohlenstoffträger (z. B. Holzkohle, Fruchtkerncarbonisat etc.) -nur langsam von Wasserdampf angegriffen wird.

Im Allgemeinen können Carbonate, Nitrate, Sulfate oder andere organische Salze als Vorstufen zur Bildung der Oberflächenoxide eingesetzt werden, die unter Einwirkung hoher Temperaturen größer 400 °C, bevorzugt jedoch bei Aktivierungstemperaturen von 500 bis 950 °C, Oxide bilden.

Der Zuschlagstoff kann weiterhin ausgewählt sein aus (Tri-)Ammoniumphosphat, (Di-)Ammoniumhydrogenphosphat, Ammoniumdihydrogenphosphat, (Tri-)Kaliumphosphat, (Di-)Kaliumhydrogenphosphat, Kaliumdihydrogenphosphat sowie deren Mischungen:
(Di-)Ammoniumhydrogenphosphat ist als Zuschlagstoff wegen der hohen Wasserlöslichkeit in einem wasserbasierten glucosehaltigen Ausgangsstoff besonders geeignet. Bei der Umsetzung des Zuschlagstoffes mit dem wasserbasierten kohlenhydrathaltigen bzw. glucosehaltigen Ausgangsstoff reagiert beispielsweise (Di-)Ammoniumhydrogenphosphat katalytisch mit dem Zucker des Bindemittels, wobei der Zucker in mehreren Reaktionsschritten aromatisiert wird. Der katalytische Effekt liegt insbesondere darin begründet, dass sich Phosphate an der OH-Gruppe des Zuckers unter Wasserabspaltung anlagern bzw. kondensieren und anschließend unter Ausbildung einer Doppelbindung im Zuckerring -letztendlich unter Aromatisierung bzw. Olefinisierung - abgespalten werden.

Wird die Aktivkohle mit Wasserdampf aktiviert, reagiert das aromatisierte Bindemittel während der Aktivierung der Formaktivkohle wesentlich schlechter mit Wasserdampf als der Kohlenstoffträger. Der Aromatisierungsprozess des Zuckers verläuft im Wesentlichen katalytisch, wobei der Ascheanteil in der Aktivkohle nicht oder nur unwesentlich ansteigt.

Beim Kohlenstoffträger handelt es sich vorzugsweise um Kohlenstoff aus nachwachsenden Rohstoffen, insbesondere um Holzkohle oder andere lignocellulosebasierte Naturstoffe. Grundsätzlich ist es jedoch auch möglich, fossile Kohlenstoffträger, insbesondere Braunkohle und/oder Braunkohlenkoks und/oder Steinkohle und/oder Mischungen aus nachwachsenden und fossilen Kohlenstoffträgern, mit dem Bindemittel zur Herstellung von Formaktivkohle zu mischen. Weiterhin können auch synthetische Polymere, beispielsweise auf Basis von Polyvinylbenzol oder ähnlicher, auch Heteroatome enthaltender synthetischer Polymere, als Kohlenstoffträger verwendet werden.

Die erfindungsgemäß eingesetzte Formaktivkohle enthält die katalytisch aktiven Komponenten bzw. Dotierungsstoffe homogen verteilt in einer kohlenstoffhaltigen Matrix. Aufgrund der während des Herstellungsprozesses herrschenden hohen Temperaturen ist davon auszugehen, dass die Dotierungsstoffe teilweise und/oder vollständig chemisch verändert werden. Beispielsweise beträgt der Dissoziationsdruck von Kaliumcarbonat gemäß dem nachfolgenden Gleichgewicht bei 1000 °C etwa 5 Torr:

K₂CO₃ ↔ K₂O + CO₂

Weiterhin ist bekannt, dass Kaliumcarbonat mit dem Kohlenstoffträger Oberflächenkomplexe bildet, die C-O-K-Fragmente enthalten. Ebenfalls wird in der Fachliteratur die Bildung von Interkalationsverbindungen postuliert, bei welchen insbesondere metallisches Kalium auf Zwischengitterplätzen einer Graphitgitterstruktur platziert ist. Röntgenstrukturanalysen von Aktivkohlen zeigen, dass Kohlenstoff nicht nur amorph, sondern auch in Form von sehr kleinen Kristallen, welche die normale Graphitgitterstruktur aufweisen, anzutreffen ist.

Es ist daher davon auszugehen - ohne sich auf diese Theorie festlegen zu wollen -, dass die Formaktivkohlen in den aus den Dotierungsstoffen entstandenen aktiven Zentren nicht mehr die ursprünglich eingesetzten Dotierungsstoffe, sondern zumindest teilweise Einheiten, insbesondere Cluster und Interkalationsverbindungen, mit anderer chemischer Struktur aufweisen. Es ist anzunehmen, dass sich die Interkalationsverbindungen nur auf die Graphitgitterstruktur beschränken.
Es ist weiterhin bekannt, dass sich Interkalationsverbindungen mit den Alkali- und Erdalkalimetallen bilden, die als sehr starke Reduktionsmittel agieren und zugleich auch an Wasserstoffspeicher- und Wasserstoffübertragungsreaktionen aktiv teilnehmen.
Für weitergehende diesbezügliche Einzelheiten zum Katalysator gemäß dieser Ausführungsform wird auf die DE 10 2004 033 561 A1 sowie die DE 10 2004 033 561 B4 verwiesen.
Gemäß einer besonderen Ausführungsform der vorliegenden Erfindung kann als Katalysator eine Formaktivkohle eingesetzt werden, wie sie in der DE 10 2006 025 450 A1 bzw. in der zu derselben Patentfamilie gehörenden WO 2007/137856 A2 beschrieben ist.

Gemäß dieser Ausführungsform kommt also als Katalysator eine Formaktivkohle zum Einsatz, welche herstellbar ist aus einer pressfähigen Masse, welche ein vermahlenes kohlenstoffhaltiges Material, ein Bindemittel und mindestens ein metallhaltiges Dotierungsreagenz enthält, und welche verpresst, getrocknet, carbonisiert und anschließend mittels eines Aktivierungsgases aktiviert wird, wobei ein erstes Dotierungsreagenz enthalten ist, wobei das erste Dotierungsreagenz ein Metallsalz ist, dessen Metall ausgewählt ist aus der Gruppe von Metallen der 3. bis 6. Hauptgruppe des Periodensystems der Elemente, Übergangsmetallen, Seltenen Erden und Halbmetallen und/oder wobei das erste Dotierungsreagenz ein Iodid der Alkali- oder Erdalkalimetalle ist, und wobei gegebenenfalls ein zweites Dotierungsreagenz der Formel M²ₚ(EO_{q})ᵣ enthalten ist, wobei M² ausgewählt ist aus Alkalimetallen und Erdalkalimetallen, E ein Element der 3. bis 7. Hauptgruppe des Periodensystems der Elemente ist und p, q und r jeweils ganze Zahlen ≥ 1 sind; dabei kann das zweite Dotierungsreagenz insbesondere ausgewählt werden aus Hydroxiden und Carbonaten.

Im Laufe der Reaktionsführung wird insbesondere beobachtet, dass der Katalysator, vermutlich durch Anlagerung von Alkoholaten, an Gewicht zunimmt. Diese Gewichtszunahme beträgt insbesondere ca. 10 bis 15 Gew.-%. Insbesondere scheinen sich auf der Katalysatoroberfläche - ohne sich auf diese Theorie festlegen zu wollen - Gleichgewichte zwischen verschiedenen adsorbierten Verbindungen, insbesondere Alkoholaten, auszubilden.

Wie oben bereits ausgeführt, dient das erfindungsgemäße Verfahren insbesondere zur Herstellung höherer Alkohole, Aldehyde, Ketone und Alkane sowie zur Herstellung von Aromaten. Im Allgemeinen werden bei Durchführung des erfindungsgemäßen Verfahrens als Produkt oder Produktgemisch Alkohole, insbesondere verzweigte, vorzugsweise verzweigte primäre Alkohole, und/oder Aldehyde und/oder Ketone und/oder Alkane und/oder Aromaten sowie deren Gemische erhalten. Was in diesem Zusammenhang die Kettenlänge bzw. die Anzahl der Kohlenstoffatome der Produkte anbelangt, so werden bei erfindungsgemäßer Verfahrensführung üblicherweise als Produkt oder Produktgemisch C₅-C₃₅-Verbindungen, insbesondere C₅-C₃₀-Verbindungen, vorzugsweise C₆-C₂₅-Verbindungen, bevorzugt C₆-C₂₀-Verbindungen erhalten.

Des Weiteren kann es im Rahmen der vorliegenden Erfindung vorgesehen sein, dass bei der Umsetzung bzw. der Verfahrensdurchführung Wasser und gegebenenfalls Wasserstoff, insbesondere Wasser und Wasserstoff, generiert werden. In diesem Zusammenhang hat es sich als besonders günstig erwiesen, wenn pro Mol an eingesetzter Verbindung (A) mindestens ein Mol Wasser generiert wird. Wie oben bereits ausgeführt, besitzt die Anwesenheit von Wasser während der Reaktionsdurchführung den Vorteil, dass die Selektivität der Produkte gezielt eingestellt bzw. verbessert wird. Dabei ist es gleichgültig, ob das Wasser aus der Reaktion stammt, in den Edukten bzw. Eduktgemischen enthalten ist oder zusätzlich eingetragen wird.

Gemäß einer besonderen Ausführungsform der vorliegenden Erfindung kann das erhaltene Produkt oder Produktgemisch nachfolgend einem Hydrierverfahrensschritt und/oder einem Hydrotreating-Verfahrensschritt, vorzugsweise einem Hydrotreating-Verfahrensschritt, unterzogen werden.

Im Rahmen üblicher Hydrierverfahren, wie beispielsweise mit Raney-Nickel, können die bei der Verfahrensdurchführung entstehenden Aldehyde und Ketone zu Alkoholen reduziert werden, wohingegen mit einem Hydrotreating-Verfahren sämtliche Verbindungen, also auch sauerstoffhaltige- und/oder stickstoffhaltige und/oder schwefelhaltige Verbindungen, zu Alkanen reduziert werden. Bei Hydrotreating handelt es sich um ein im Zuge der Erdölraffination standardmäßig durchgeführtes Verfahren, welches dem Fachmann an sich bekannt ist und folglich keiner näheren Erläuterung bedarf.

Eine weitere Besonderheit des erfindungsgemäßen Verfahrens ist darin zu sehen, dass durch die spezifische Auswahl an Edukten bzw. Eduktgemischen, wie beispielsweise Mischungen verschiedener Alkohole und Ketone mit jeweils bekannter Kettenlänge bzw. bekannter Zusammensetzung der Gemische, besondere statistische Produktverteilungen erhalten und insbesondere gezielt eingestellt werden können. Auf diese Weise ist es möglich, spezielle Produktgemische mit einer vorher festgelegten, spezifischen statistischen Produktverteilung zu erhalten.

Bei den hydrierten Produkten bzw. Produktgemischen handelt es sich insbesondere um Verbindungen, die bevorzugt als Brenn- bzw. Kraftstoffe eingesetzt werden können. So kann etwa bei einer allein mit Butanol durchgeführten Kondensation durch nachfolgendes Hydrieren aller Aldehyde, welche bei dieser Reaktion als Nebenprodukte entstehen, bei moderaten Bedingungen (200 °C, 30 bar, Raney-Nickel-Katalysator) und vorzugsweise unter Verwendung des im System gebildeten Wasserstoffs und nach Abtrennung von Wasser durch Phasentrennung eine Alkohol/Alkan-Fraktion gewonnen werden, die als motortaugliches Benzin genutzt werden kann.

Beim Einsatz von längerkettigen Alkoholen, wie etwa Butanolen, Pentanolen, Hexanolen oder höheren Alkoholen oder deren Gemischen, als alleinigen Edukten, jeweils mit oder ohne Ethanolanteile, kann das entstehende Reaktionsgemisch, gegebenenfalls nach vorheriger Abtrennung leicht siedender Anteile, direkt durch sogenanntes Hydrotreating in einem Schritt in eine nur Alkane enthaltene Kerosinfraktion umgewandelt werden. Die so erhältlichen Produkte sind grundsätzlich in ihren physikalischen Eigenschaften nicht von fossil erzeugtem und JET-A1 konformem Flugzeugkerosin zu unterscheiden. Bedingt durch den möglichen hohen Anteil von Alkanen in einem mit dem erfindungsgemäßen Verfahren erhältlichen Produktgemisch kann eine erhebliche Menge sowohl an Wasserstoff als auch an Reaktorvolumen bei einem anschließenden Hydrotreating des Gemisches zu einem nur aus Alkanen bestehendem Produkt, wie etwa Jetfuel, eingespart werden. Durch die gezielte Auswahl der Kettenlänge z. B. der Alkohole und deren Art, d. h. primär, sekundär, verzweigter oder n-Alkohol, sowie beliebiger Gemische daraus kann gezielt die gewünschte Siedepunkts- und Kettenlängenverteilung der Kerosinfraktion eingestellt bzw. beeinflusst werden. Weiterhin ergeben sich auf diese Weise Kostenvorteile durch eine Zeit- und Energieeinsparung, da nicht ein Gemisch mit einer weiten Verteilung der Siedepunkte aufwendig destilliert werden muss, wie dies beispielsweise bei über Fischer-Tropsch-Synthesen hergestellten Produkten mit breiter statistischer Kohlenstoffkettenverteilungen üblich ist.
Weiterhin kann das Gemisch aus Alkoholen und Alkanen einer katalytischen Dehydratisierung, gefolgt von einer Hydrierung, unterworfen werden, um eine nur Alkane enthaltene Mischung, die für Jetfuel geeignet ist, zu erhalten.

Weitere Ausgestaltungen, Abwandlungen und Variationen sowie Vorteile der vorliegenden Erfindung sind für den Fachmann beim Lesen der Beschreibung ohne weiteres erkennbar und realisierbar, ohne dass er dabei den durch die Ansprüche definierten Rahmen der vorliegenden Erfindung verlässt.

Die vorliegende Erfindung wird anhand der folgenden Ausführungsbeispiele veranschaulicht, welche die vorliegende Erfindung jedoch keinesfalls beschränken.

### Ausführungsbeispiele:

### Eingesetzte Katalysatorsysteme

In den vorangehenden Ausführungsbeispielen kommen als Katalysatoren alkali- und erdalkalidotierte, mittels Phosphat basisch eingestellte Aktivkohlesubstrate zum Einsatz, wie sie gemäß DE 10 2004 033 561 A1 erhalten werden. Die eingesetzten Katalysatorsysteme "Kat1", Kat2" und "Kat3" sind nachfolgend näher chemisch charakterisiert:
Chemische Zusammensetzung der eingesetzten Katalysatoren, bezogen auf Asche:

| | | | |
|---|---|---|---|
| Kat1: | Aschegehalt | 24,4 | Gew.-% |
| | Natrium | 7,28 | g/kg |
| | Kalium | 238 | g/kg |
| | Magnesium | 11,7 | g/kg |
| | Phosphor | 19,2 | g/kg |
| | Calcium | 167 | g/kg |
| | Summe | 443,18 | g/kg |
| | | | |
| Kat2: | Aschegehalt | 18,1 | Gew.-% |
| | Natrium | 10,7 | g/kg |
| | Kalium | 360 | g/kg |
| | Magnesium | 13,9 | g/kg |
| | Phosphor | 36,5 | g/kg |
| | Calcium | 110 | g/kg |
| | Summe | 531,1 | g/kg |
| | | | |
| Kat3: | Aschegehalt | 11,0 | Gew.-% |
| | Natrium | 22,2 | g/kg |
| | Kalium | 307 | g/kg |
| | Magnesium | 11,8 | g/kg |
| | Phosphor | 5,6 | g/kg |
| | Calcium | 130 | g/kg |
| | Summe | 476,6 | g/kg |

Molare chemische Zusammensetzung der getesteten Katalysatoren:

| | | | | | | | | | |
|---|---|---|---|---|---|---|---|---|---|
| Kat1 | Na | K | Mg | Ca | P | 0,5 | Na/P | 0,8 | Mg/P |
| | 0,3 | 6,1 | 0,5 | 4,2 | 0,6 | 9,8 | K/P | 6,7 | Ca/P |
| Kat2 | Na | K | Mg | Ca | P | 0,4 | Na/P | 0,5 | Mg/P |
| | 0,5 | 9,2 | 0,6 | 2,7 | 1,2 | 7,8 | K/P | 2,3 | Ca/P |
| Kat3 | Na | K | Mg | Ca | P | 5,3 | Na/P | 2,7 | Mg/P |
| | 1,0 | 7,9 | 0,5 | 3,2 | 0,2 | 43,5 | K/P | 18,0 | Ca/P |

### Versuchsdurchführung

Erfindungsgemäß hergestellte Pellets aus Formaktivkohle (vgl. vorstehende Beschreibung der eingesetzten Katalysatorsysteme) werden mechanisch zerkleinert und die Bruchstücke (1-2 mm Fraktion) als Katalysatoren in einen Edelstahlreaktor eingefüllt. Der Reaktor hat einen inneren Durchmesser von 21 mm und ein Volumen von 75 ml. Die eingefüllte Masse beträgt 30 g.

Als Ausgangsstoffe bzw. Reaktanden kommen sowohl wasserfreie als auch wässrige, lineare und verzweigte Alkohole in Betracht. Ebenso sind Mischungen verschiedener Alkohole, verschiedener Aldehyde und Ketone möglich.

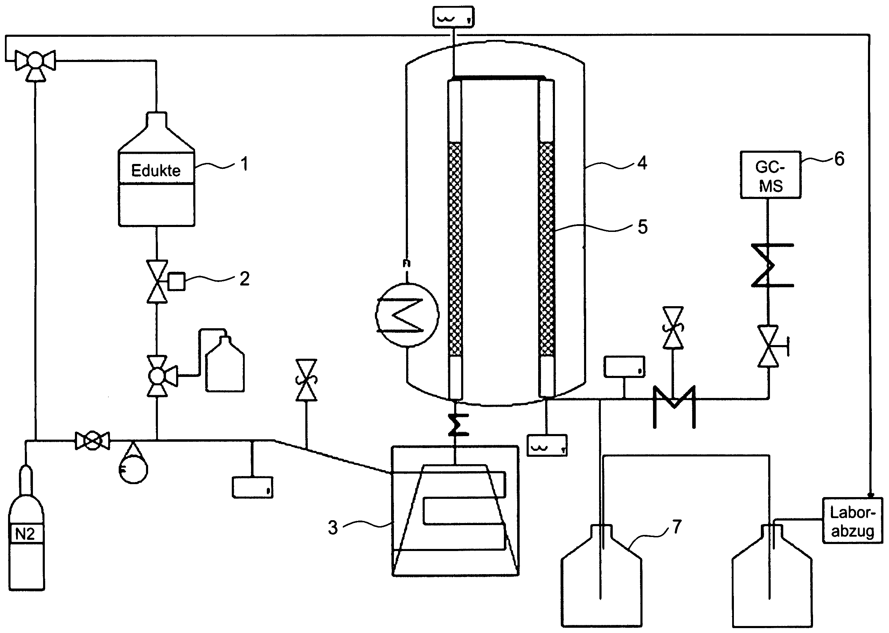

Eine schematische Darstellung des verwendeten Versuchsaufbaus ist in der einzigen Figur abgebildet: Die Reaktanden befinden sich in einem Druckbehälter 1 und werden über einen kalibrierten Massendurchflussregler 2 dosiert. Vor dem Eintritt in den Verdampfer kann optional ein Inertgas, im Allgemeinen Stickstoff, hinzugegeben werden. Der Volumenstrom wird über ein Nadelventil mit einem Schwebkörperdurchflussmesser eingestellt und liegt typischerweise zwischen 0 und 40 Nl/h. In einem elektrisch beheizten Rohr 3 werden die Reaktanden verdampft und auf bis zu 345 °C oder wahlweise auch mehr erhitzt. Das Inertgas wird so ebenfalls auf bis zu 345 °C oder wahlweise auch mehr erwärmt. Das gasförmige Gemisch strömt vom Verdampfer in den Reaktor 4, wo an der Oberfläche des Katalysators die Reaktion abläuft. Typische Kontaktzeiten betragen zwischen 0,01 und 30 Sekunden, wenn die Reaktion in der Gasphase abläuft. Nach dem Reaktor wird das Produktgemisch kondensiert 7 und der bei Raumtemperatur nicht kondensierbare Anteil gasförmig abgeführt. Über eine beheizte Leitung ist eine direkte Probennahme aus dem Produktgasstrom möglich.

Die Zusammensetzung der flüssigen Produkte wird mit einer HPLC 1200 mit RI-Detektor und einer Rezex ROA, 300 x 7,8 mm Säule von Phenomenex der Firma Agilent und der Wassergehalt der Probe mittels Karl-Fischer-Titration bestimmt. Zur Bestimmung weiterer Produkte wird ein GC/MS+FID 6890N/5975 mit einer DB-FFAP, 30 m x 0,25 mm x 0,25 µm Säule von der Firma Agilent genutzt.

Ausgewählte Versuchsergebnisse sind in den nachfolgenden Tabellen 1 bis 4 wiedergegeben. Abgesehen von den dort aufgeführten Verbindungen findet sich noch hauptsächlich Wasser aus der Reaktion im Produkt.

Neben der flüssigen Phase wird auch die Zusammensetzung der entstehenden gasförmigen Produkte bestimmt. Die Analysen zeigen, dass hier Wasserstoff und Kohlenstoffdioxid die Hauptanteile (in Mol-%) bilden. Weitere Komponenten sind Alkane und Alkene verschiedener Kettenlängen. Insgesamt entsteht, speziell bei Temperaturen unter 350 °C, jedoch nur ein sehr geringer Anteil an gasförmigen Produkten (< 5 Gew.-%). Mit steigender Temperatur oder Verweilzeit kann der Anteil allerdings abhängig von den eingesetzten Materialien schnell ansteigen.

Um auch Versuche unter erhöhtem Druck durchzuführen, wurde eine Anlage genutzt, in welcher das Edukt mittels einer HPLC-Pumpe aus einer Vorlage in einen mit Öl auf 300 °C beheizten Verdampfer gefördert wird. Die Reaktion läuft in einem auf bis zu 345 °C oder mehr elektrisch beheizten Reaktor (mit einem Volumen von 120 ml) an 50 g des beschriebenen Katalysators ab. Das Produkt wird nach der Reaktion in zwei wassergekühlten Wärmetauschern kondensiert. Mit dieser Anlage wurden Versuche bei Drücken bis zu 60 bar (absolut) durchgeführt. Ein Trägergas wird nicht verwendet. Die Ergebnisse dieser Versuche sind gleichfalls in Tabelle 1 wiedergegeben und dort als Flüssigphasenversuch (Versuch Nr. 6) gekennzeichnet.

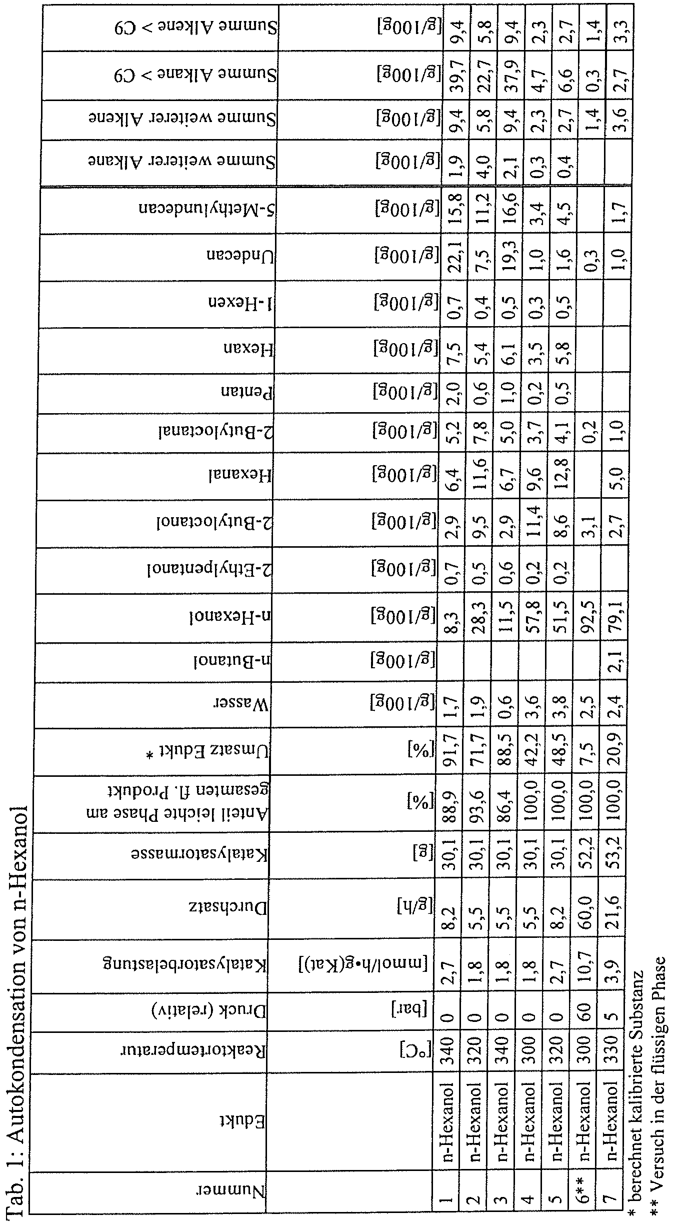

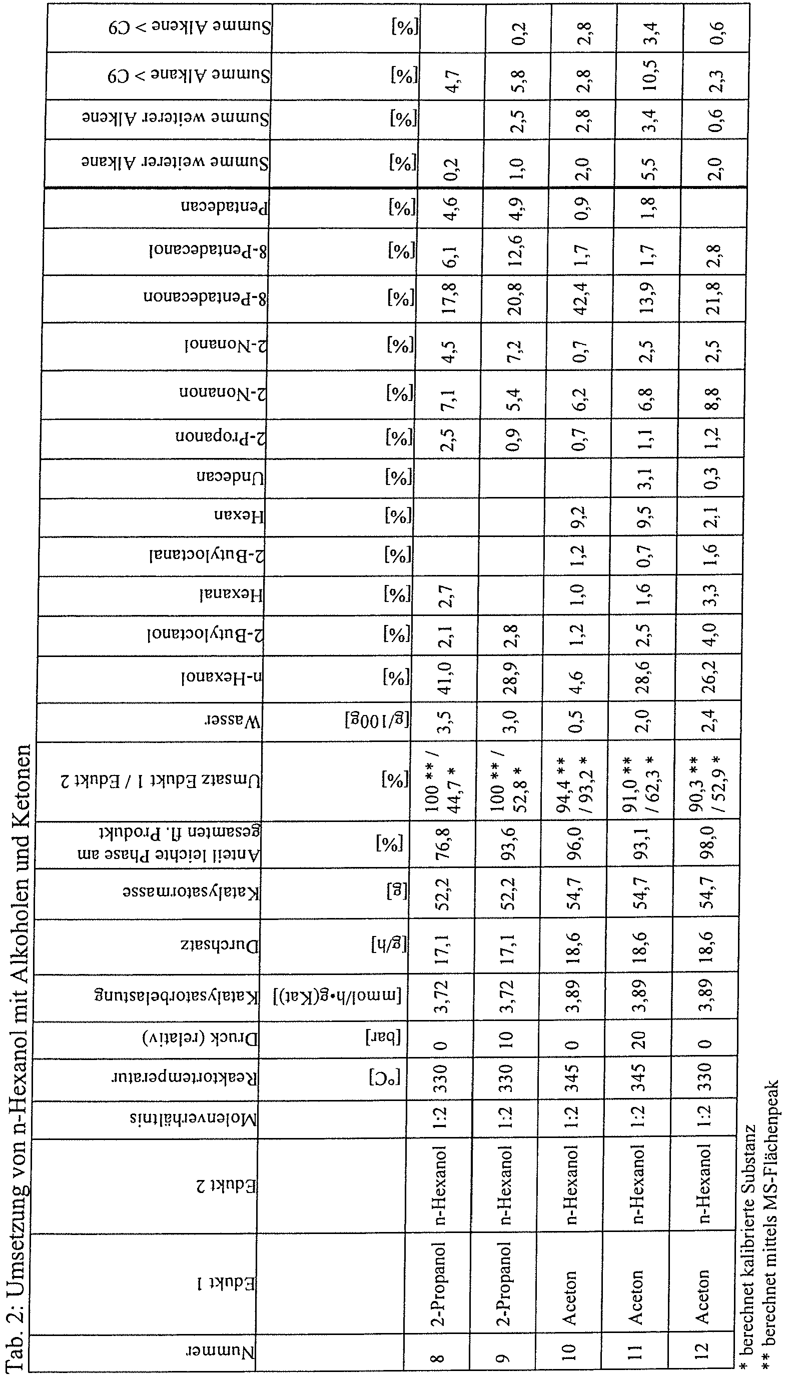

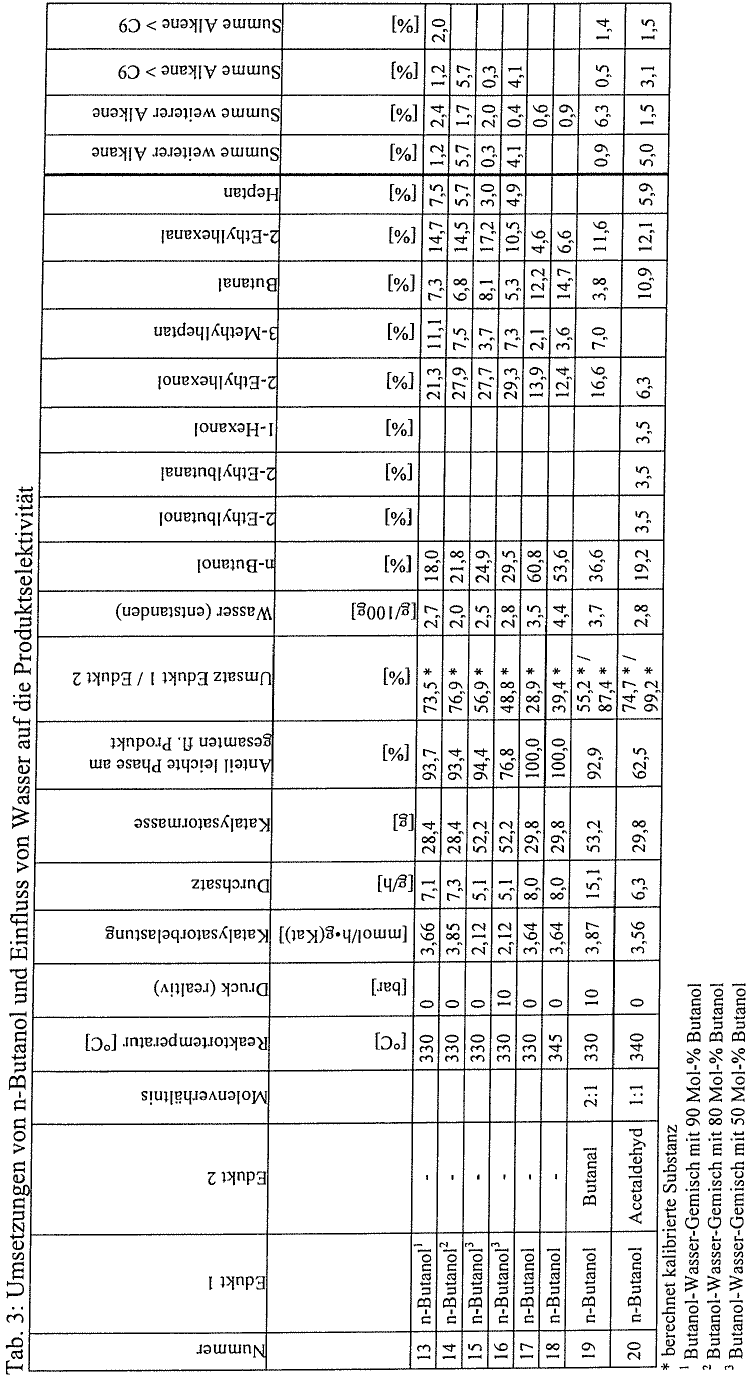

**Tab. 4: Kalibrierte Werte aus den Versuchen 8 bis 20**

| Nummer | 8 | 9 | 10 | 11 | 12 | 13 | 14 | 15 | 16 | 17 | 18 | 19 | 20 |
|---|---|---|---|---|---|---|---|---|---|---|---|---|---|
| n-Butanol [g/100g] | | | | | | 25,8 | 32,4 | 34,7 | 41,2 | 71,1 | 60,7 | 36,6 | 19,2 |
| n-Butanol [%] | | | | | | 18,0 | 21,8 | 24,9 | 29,5 | 60,8 | 53,6 | 30,0 | 15,9 |
| n-Hexanol [g/100g] | 42,7 | 36,5 | 4,1 | 22,7 | 28,5 | | | | | | | | |
| n-Hexanol [%] | 41,0 | 28,9 | 4,6 | 28,6 | 26,2 | | | | | | | | |
| 2-Ethylhexanol [g/100g] | | | | | | 12,9 | 17,3 | 15,3 | 16,6 | 5,4 | 4,8 | | |
| 2-Ethylhexanol [%] | | | | | | 21,3 | 27,9 | 27,7 | 29,3 | 13,9 | 12,4 | | |
| Butanal [g/100g] | | | | | | 10,5 | 10,0 | 11,6 | 5,6 | 13,7 | 17,5 | | |
| Butanal [%] | | | | | | 7,3 | 6,8 | 8,1 | 5,3 | 12,2 | 14,7 | | |
| Hexanal [g/100g] | 2,7 | | | 1,1 | | | | | | | | | |
| Hexanal [%] | 2,7 | | | 1,6 | | | | | | | | | |
| 3-Methylheptan [g/100g] | | | | | | 5,6 | 3,4 | | 2,8 | | | | |
| 3-Methylheptan [%] | | | | | | 11,1 | 7,5 | | 7,3 | | | | |

Alle in den Tabellen 1 bis 4 angegebenen Werte beziehen sich auf die leichte Phase, welche in den durchgeführten Versuchen üblicherweise mindestens 92 Gew.-% der Probe ausmacht. Die schwere Phase besteht zu mehr als 90 Gew.-% aus Wasser und wurde nicht weiter analysiert.

In den Tabellen 2 und 3 beziehen sich die Werte auf Flächenprozent, d. h. den Anteil der Peakfläche des betreffenden Peaks im Verhältnis zur Summe aller automatisch identifizierten Peaks des Chromatogramms. Als Detektor wurde ein Massenspektrometer verwendet.

Die entsprechenden kalibrierten Werte, welche durch Vergleich mit der jeweiligen Reinsubstanz ermittelt wurden, zu den Versuchen 8 bis 20 zeigt Tabelle 4.

In einem weiteren Versuch (Versuch 21) wurde mit der zuvor beschriebenen Apparatur die Kopplung bzw. Kondensation von Produkten der ABE-Fermentation untersucht. Bei der ABE-Fermentation entstehen durch Fermentation Produktgemische, die insbesondere Aceton, Butanol und Ethanol im molaren Verhältnis von etwa 3:6:1 im wässrigen Medium enthalten. Derartige ABE-Fermentationsprodukte mit den entsprechenden Anteilen an Aceton, Butanol und Ethanol sind beispielsweise kommerziell verfügbar.

Im Rahmen der durchgeführten Untersuchungen wurden jedoch keine Fermentationsprodukte eingesetzt, sondern Mischungen der Einzelsubstanzen. Die molare Verhältnisse des Eduktgemisches betragen im vorliegenden Fall Aceton : Butanol : Ethanol (A:B:E) 3 : 6 : 1. Diese molaren Eduktverhältnisse ergeben sich bei Versuch 21a (leichte Produktphase) und 21b (schwere Produktphase) aus der gängigen Produktzusammensetzung der ABE-Fermentation.

Zusätzlich wurden 10 Massenprozent Wasser hinzugegeben, da bei der ABE-Fermentation eine wässrige Lösung entsteht.

Die Ergebnisse dieser Versuche sind in der nachfolgenden Tabelle 5 zusammengefasst. Im Unterschied zu den in den Tabellen 1 bis 4 wiedergegebenen Versuchen wurde in Versuch 21 sowohl die leichte Phase als auch die schwere Phase analysiert.

Die Bildung der Hauptprodukte der Kondensation der ABE-Fermentationsprodukte wird anhand der folgenden Gleichungen verdeutlicht, wobei Hexanol (HexOH) durch Kondensation von Butanol (BuOH) und Ethanol (EtOH) entsteht:

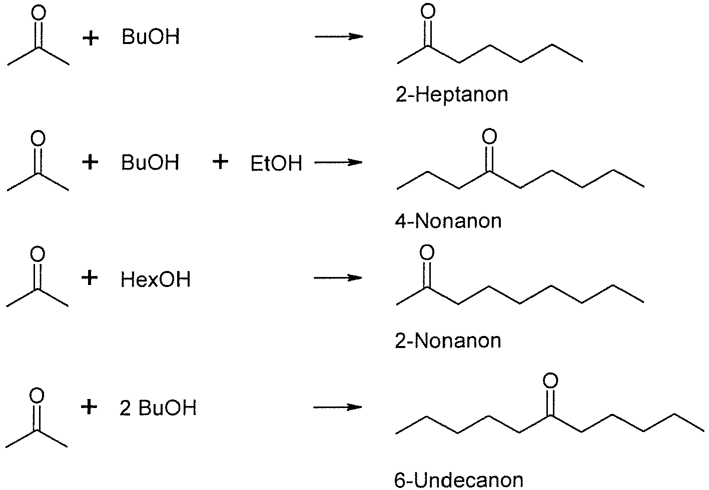

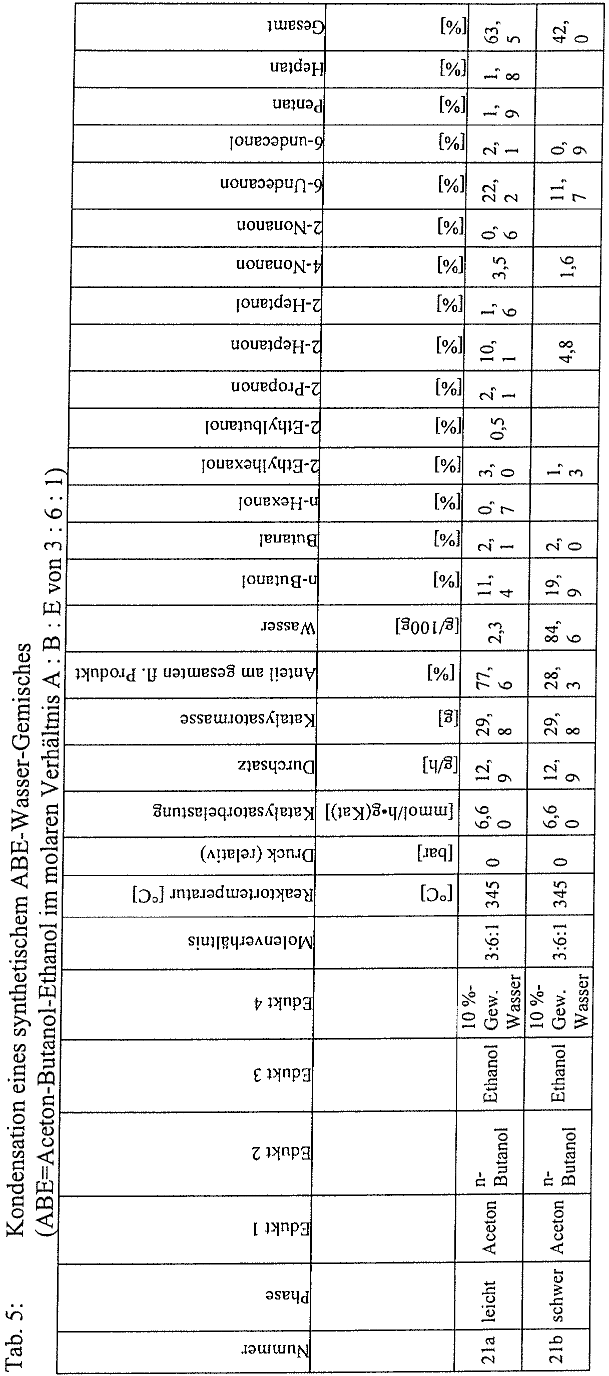

## Patentansprüche

1. Verfahren zur katalytischen Kondensation und/oder Kopplung von mindestens eine Oxo- und/oder Hydroxylfunktion enthaltenden organischen Verbindungen mit CH-aciden Verbindungen zur Erzeugung höherer Alkohole, Aldehyde, Ketone, Aromaten und/oder Alkane sowie deren Gemische, wobei als Eduktgemisch
(A) mindestens eine mindestens eine Oxo- und/oder Hydroxylfunktion enthaltende organische Verbindung, welche mindestens 3 Kohlenstoffatome aufweist und ausgewählt ist aus der Gruppe von primären und/oder sekundären Alkoholen, Aldehyden und Ketonen sowie deren Mischungen,
mit
(B) mindestens einer CH-aciden Verbindung, welche mindestens 3 Kohlenstoffatome aufweist, wobei die CH-acide Verbindung (B) ausgewählt ist aus der Gruppe von primären und/oder sekundären Alkoholen, Carbonsäuren, Carbonsäureanhydriden, Carbonsäureestern, Aldehyden, Ketonen, Nitrilen, Nitroverbindungen, organischen Nitraten und deren Mischungen,
wobei (A) und (B) identisch sein können,
eingesetzt und in Gegenwart mindestens eines Katalysators umgesetzt wird, wobei der Katalysator ein Aktivkohlesubstrat, welches mit mindestens einem Metall versehen ist, umfasst,
wobei als Katalysator eine Formaktivkohle zum Einsatz kommt, welche herstellbar ist durch ein Verfahren zur Herstellung von Formaktivkohle aus einem Kohlenstoffträger, einem Bindemittel und einer katalytischen Komponente der allgemeinen Formel (I)
[M]ₘ₃[AOₙ₄]ₘ₄ (I)
wobei
- M ein Kation bezeichnet und ausgewählt ist aus der Gruppe von Alkali- oder Erdalkalimetallkationen;
- m3 und m4 stöchiometrische Koeffizienten mit ganzen Zahlen mit m3 ≥ 1 und m4 ≥ 1 bezeichnen;
- [AOₙ₄] ein sauerstoffhaltiges Anion mit dem ganzzahligen stöchiometrischen Koeffizienten n4 ≥ 1 bezeichnet;
- [AOₙ₄] vorzugsweise ausgewählt wird aus der Gruppe von Carbonaten oder Hydroxiden,
wobei das Bindemittel erhalten wird aus der Umsetzung eines wasserlöslichen kohlenhydrathaltigen Ausgangsstoffes mit einem Glucosegehalt von ≥ 50 Gew.-%, wobei der Kohlenstoffträger zunächst mit der katalytischen Komponente vermischt wird, wobei anschließend die Mischung aus katalytischer Komponente und Kohlenstoffträger mit dem Bindemittel vermischt wird, wobei die so erhaltene Mischung aus Kohlenstoffträger, katalytischer Komponente und Bindemittel zu Formlingen verpresst wird und wobei die Formlinge carbonisiert und aktiviert werden, wobei das Bindemittel aus der Umsetzung des kohlenhydrathaltigen Ausgangsstoffes mit einem Zuschlagstoff erhalten wird, wobei zum Erhalt des Bindemittels der Zuschlagstoff dem kohlenhydrathaltigen Ausgangsstoff zugegeben wird vor der Vermischung des Bindemittels mit der Mischung aus dem Kohlenstoffträger und der katalytischen Komponente, wobei der Zuschlagstoff ausgewählt ist aus der Gruppe von Phosphorsäuren und/oder deren Salzen, Schwefelsäuren und/oder deren Salzen und/oder Schwefelsäurederivaten und/oder deren Salzen.

2. Verfahren nach Anspruch 1, **dadurch gekennzeichnet,**
**dass** die mindestens eine Oxo- und/oder Hydroxylfunktion enthaltende organische Verbindung (A) ausgewählt ist aus C₃-C₂₅-Verbindungen, insbesondere C₃-C₂₀-Verbindungen, vorzugsweise C₃-C₁₅-Verbindungen, bevorzugt C₃-C₁₀-Verbindungen, besonders bevorzugt C₃-C₈-Verbindungen, sowie Gemischen verschiedener Verbindungen mit der vorgenannten Kohlenstoffanzahl; und/oder
**dass** die mindestens eine Oxo- und/oder Hydroxylfunktion enthaltende organische Verbindung (A) ausgewählt ist aus linearen oder verzweigten Verbindungen.

3. Verfahren nach Anspruch 1 oder 2, **dadurch gekennzeichnet,**
**dass** die CH-acide Verbindung (B) ausgewählt ist aus C₃-C₂₅-Verbindungen, insbesondere C₃-C₂₀-Verbindungen, vorzugsweise C₃-C₁₅-Verbindungen, bevorzugt C₃-C₁₀-Verbindungen, besonders bevorzugt C₃-C₈-Verbindungen, sowie Gemischen verschiedener Verbindungen mit der vorgenannten Kohlenstoffanzahl; und/oder Unser Zeichen: 14.6037.1 as Amtliches Aktenzeichen.: EP 12 806 350.0-1451 / 2 791 086 22. Januar 2018
**dass** die CH-acide Verbindung (B) ausgewählt ist aus der Gruppe von primären und/oder sekundären Alkoholen, Carbonsäureanhydriden, Carbonsäureestern, Aldehyden, Ketonen, Nitrilen, Nitroverbindungen, organischen Nitraten und deren Mischungen, vorzugsweise aus der Gruppe von primären und/oder sekundären Alkoholen, Aldehyden, Ketonen und deren Mischungen.

4. Verfahren nach einem der vorangehenden Ansprüche, **dadurch gekennzeichnet, dass** die CH-acide Verbindung (B) ausgewählt ist aus
primären und/oder sekundären Alkoholen der allgemeinen Formel (I) 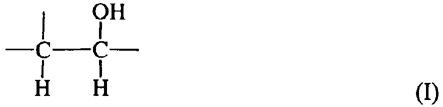 und/oder
Nitroverbindungen und/oder organischen Nitraten der allgemeinen Formel (II) 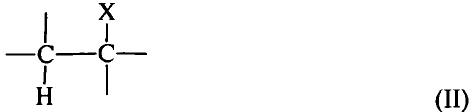 mit X = NO₂, ONO₂
und/oder
Carbonylverbindungen der allgemeinen Formel (III) 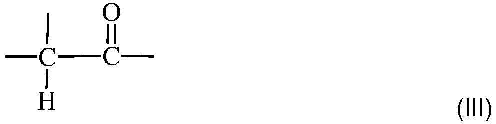 und/oder Unser Zeichen: 14.6037.1 as Amtliches Aktenzeichen.: EP 12 806 350.0-1451 / 2 791 086 22. Januar 2018
Nitrilverbindungen der allgemeinen Formel (IV)

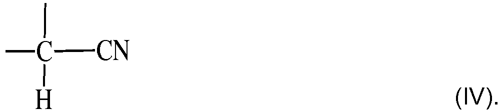

5. Verfahren nach einem der vorangehenden Ansprüche, **dadurch gekennzeichnet,**
**dass** das Verfahren in der Gasphase und/oder in der Flüssigphase und/oder im überkritischen Bereich, vorzugsweise in der Gasphase, durchgeführt wird; und/oder
**dass** das Verfahren oberhalb der Siedetemperaturen der Edukte und/oder Produkte, vorzugsweise oberhalb der Siedetemperaturen der Edukte und Produkte, durchgeführt wird; und/oder
**dass** das Verfahren bei Temperaturen im Bereich von 150 °C bis 600 °C, insbesondere 250 bis 450 °C, vorzugsweise 300 bis 400 °C, durchgeführt wird; und/oder
**dass** das Verfahren bei reduziertem Druck, bei Atmosphärendruck oder bei erhöhtem Druck, vorzugsweise bei Atmosphärendruck oder bei erhöhtem Druck, durchgeführt wird, insbesondere bei einem Absolutdruck im Bereich von Atmosphärendruck bis 150 bar, insbesondere im Bereich von Atmosphärendruck bis 80 bar, vorzugsweise im Bereich von Atmosphärendruck bis 25 bar.

6. Verfahren nach einem der vorangehenden Ansprüche, **dadurch gekennzeichnet, dass** das Verfahren mit Reaktionsdauern und/oder Kontaktzeiten im Bereich von 0,01 Minuten bis 12 Stunden, insbesondere 0,1 Minuten bis 10 Stunden, vorzugsweise 1 Minute bis 5 Stunden, durchgeführt wird, insbesondere wobei das Verfahren in der Gasphase und/oder im überkritischen Bereich mit Reaktionsdauern und/oder Kontaktzeiten im Bereich von 0,001 bis 120 Sekunden, insbesondere 0,01 bis 60 Sekunden, vorzugsweise 0,05 bis 30 Sekunden, durchgeführt wird und/oder insbesondere wobei das Verfahren in der Flüssigphase mit Reaktionsdauern und/oder Kontaktzeiten im Bereich von 0,01 Minuten bis 12 Stunden, insbesondere 0,1 Minuten bis 10 Stunden, vorzugsweise 1 Minute bis 5 Stunden, durchgeführt wird.
Unser Zeichen: 14.6037.1 as Amtliches Aktenzeichen.: EP 12 806 350.0-1451 / 2 791 086 22. Januar 2018

7. Verfahren nach einem der vorangehenden Ansprüche, **dadurch gekennzeichnet, dass** das Verfahren in Gegenwart von Wasser, vorzugsweise in Form von Wasserdampf, und/oder in Gegenwart von Wasserstoff durchgeführt wird.

8. Verfahren nach einem der vorangehenden Ansprüche, **dadurch gekennzeichnet,**
**dass** das Verfahren mit einer Raum-Zeit-Ausbeute, angegeben als Menge aller gebildeter Produkte pro Katalysatorvolumen und pro Zeiteinheit, im Bereich von 10 bis 3.000 g/(Liter • h), insbesondere 25 bis 2.500 g/(Liter • h), vorzugsweise 30 bis 2.000 g/(Liter • h), besonders bevorzugt 50 bis 1.500 g/(Liter • h), durchgeführt wird; und/oder
**dass** das Verfahren mit einer Raumgeschwindigkeit, angegeben als Stoffmenge aller gebildeter Produkte pro Katalysatormasse und pro Zeiteinheit, im Bereich von 0,1 bis 100 mol/(kg • h), insbesondere 0,5 bis 25 mol/(kg • h), vorzugsweise 1,0 bis 20 mol/(kg • h), besonders bevorzugt 1,25 bis 18 mol/(kg • h), ganz besonders bevorzugt 1,5 bis 15 mol/(kg • h), durchgeführt wird.

9. Verfahren nach einem der vorangehenden Ansprüche, **dadurch gekennzeichnet,**
**dass** als Produkt oder Produktgemisch Alkohole, insbesondere verzweigte, vorzugsweise verzweigte primäre Alkohole, und/oder Aldehyde und/oder Ketone und/oder Alkane und/oder Aromaten sowie deren Gemische erhalten werden, insbesondere wobei als Produkt oder Produktgemisch C₅-C₃₅-Verbindungen, insbesondere C₅-C₃₀-Verbindungen, vorzugsweise C₆-C₂₅-Verbindungen, bevorzugt C₆-C₂₀-Verbindungen erhalten werden; und/oder
**dass** bei der Umsetzung Wasser und gegebenenfalls Wasserstoff, insbesondere Wasser und Wasserstoff, generiert werden, insbesondere wobei pro Mol an eingesetzter Verbindung (A) mindestens ein Mol Wasser generiert wird.

10. Verfahren nach einem der vorangehenden Ansprüche, **dadurch gekennzeichnet, dass** das erhaltene Produkt oder Produktgemische nachfolgend einem Hydrierverfahrensschritt und/oder einem Hydrotreating-Verfahrensschritt, vorzugsweise einem Hydrotreating-Verfahrensschritt, unterzogen wird.

## Claims

1. A method for the catalytic condensation and/or coupling of organic compounds containing at least one oxo and/or hydroxyl function with CH-acidic compounds for the production of higher alcohols, aldehydes, ketones, aromatic compounds and/or alkanes, as well as mixtures thereof, wherein the following mixture of educts is used:
(A) at least one organic compound containing at least one oxo and/or hydroxyl function which contains at least 3 carbon atoms and is selected from the group formed by primary and/or secondary alcohols, aldehydes and ketones, as well as mixtures thereof,
with
(B) at least one CH-acidic compound which contains at least 3 carbon atoms, wherein the CH-acidic compound (B) is selected from the group formed by primary and/or secondary alcohols, carboxylic acids, carboxylic acid anhydrides, carboxylic acid esters, aldehydes, ketones, nitriles, nitro compounds, organic nitrates and mixtures thereof,
wherein (A) and (B) may be identical,
and is reacted in the presence of at least one catalyst, wherein the catalyst comprises an activated carbon substrate which is provided with at least one metal,
wherein a shaped activated carbon is used as the catalyst, which can be produced by means of a method for the production of shaped activated carbon from a carbon substrate, a binder and a catalytic component with general formula (I) :
[M]ₘ₃[AOₙ₄]ₘ₄ (I)
wherein
- M represents a cation and is selected from the group formed by alkali or alkaline earth metal cations;
- m3 and m4 represent whole-number stoichiometric coefficients with m3 ≥ 1 and m4 ≥ 1;
- [AOₙ₄] represents an oxygen-containing anion with the whole-number stoichiometric coefficient n4 ≥ 1;
- [AOₙ₄] is preferably selected from the group formed by carbonates or hydroxides,
wherein the binder is obtained by reacting a watersoluble carbohydrate-containing starting material with a glucose content of ≥ 50% by weight, wherein the carbohydrate substrate is initially mixed with the catalytic component, wherein subsequently, the mixture of the catalytic component and the carbohydrate substrate is mixed with the binder, wherein the mixture of the carbohydrate substrate, catalytic component and binder obtained in this manner is compacted into shaped bodies and wherein the shaped bodies are carbonized and activated, wherein the binder is obtained by reacting the carbohydrate-containing starting material with an additional material, wherein in order to obtain the binder, the additional material is added to the carbohydrate-containing starting material before mixing the binder with the mixture of the carbohydrate substrate and the catalytic component, wherein the additional material is selected from the group formed by phosphoric acids and/or salts thereof, sulphuric acids and/or salts thereof, and/or sulphuric acid derivatives and/or salts thereof.

2. The method as claimed in claim 1, **characterized in that**:
the organic compound containing at least one oxo and/or hydroxyl function (A) is selected from C₃-C₂₅ compounds, in particular from C₃-C₂₀ compounds, preferentially from C₃-C₁₅ compounds, preferably from C₃-C₁₀ compounds, particularly preferably from C₃-C₈ compounds, as well as mixtures of different compounds with the said numbers of carbons; and/or
the organic compound containing at least one oxo and/or hydroxyl function (A) is selected from linear or branched compounds.

3. The method as claimed in claim 1 or claim 2, **characterized in that**:
the CH-acidic compound (B) is selected from C₃-C₂₅ compounds, in particular from C₃-C₂₀ compounds, preferentially from C₃-C₁₅ compounds, preferably from C₃-C₁₀ compounds, particularly preferably from C₃-C₈ compounds, as well as mixtures of different compounds with said numbers of carbons; and/or
the CH-acidic compound (B) is selected from the group formed by primary and/or secondary alcohols, carboxylic acid anhydrides, carboxylic acid esters, aldehydes, ketones, nitriles, nitro compounds, organic nitrates and mixtures thereof, preferably from the group formed by primary and/or secondary alcohols, aldehydes, ketones, and mixtures thereof.

4. The method as claimed in one of the preceding claims, **characterized in that** the CH-acidic compound (B) is selected from:
primary and/or secondary alcohols with general formula (I): 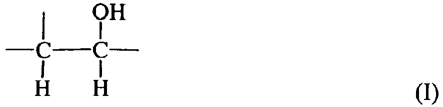 and/or
nitro compounds and/or organic nitrates with general formula (II): 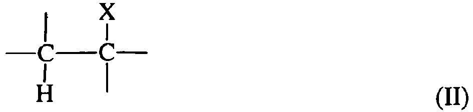 in which X = NO₂, ONO₂
and/or
carbonyl compounds with general formula (III): 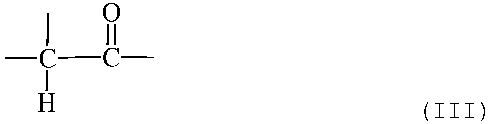 and/or
nitrile compounds with general formula (IV):

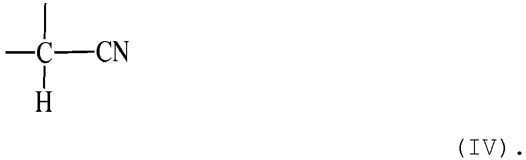

5. The method as claimed in one of the preceding claims, **characterized in that**:
the method is carried out in the gas phase and/or in the liquid phase and/or in the supercritical range; preferably in the gas phase; and/or
the method is carried out above the boiling points of the educts and/or products, preferably above the boiling points of the educts and products; and/or
the method is carried out at temperatures in the range 150°C to 600°C, in particular 250°C to 450°C, preferably in the range 300°C to 400°C; and/or
the method is carried out at a reduced pressure, at atmospheric pressure or at a raised pressure, preferably at atmospheric pressure or at a raised pressure, in particular at an absolute pressure in the range from atmospheric pressure to 150 bar, in particular in the range from atmospheric pressure to 80 bar, preferably in the range from atmospheric pressure to 25 bar.

6. The method as claimed in one of the preceding claims, **characterized in that** the method is carried out with reaction times and/or contact times in the range from 0.01 minutes to 12 hours, in particular 0.1 minutes to 10 hours, preferably 1 minute to 5 hours; in particular, when the method is carried out in the gas phase and/or in the supercritical range, the reaction times and/or contact times are in the range 0.001 to 120 seconds, in particular 0.01 to 60 seconds, preferably 0.05 to 30 seconds; and/or in particular, when the method is carried out in the liquid phase, the reaction times and/or contact times are in the range 0.01 minutes to 12 hours, in particular 0.1 minutes to 10 hours, preferably 1 minute to 5 hours.

7. The method as claimed in one of the preceding claims, **characterized in that** the method is carried out in the presence of water, preferably in the form of steam, and/or in the presence of hydrogen.

8. The method as claimed in one of the preceding claims, **characterized in that**:
the method is carried out with a space-time yield, reported as the quantity of all of the products formed per volume of catalyst and per unit of time, in the range from 10 to 3000 g/(litre • h), in particular 25 to 2500 g/(litre • h), preferably 30 to 2000 g/(litre • h), particularly preferably 50 to 1500 g/(litre • h); and/or
the method is carried out at a space velocity, reported as the quantity of all of the products formed per mass of catalyst and per unit of time, in the range from 0.1 to 100 mol/(kg • h), in particular 0.5 to 25 mol/(kg • h), preferably in the range 1.0 to 20 mol/(kg • h), particularly preferably in the range 1.25 to 18 mol/(kg • h), more particularly preferably in the range 1.5 to 15 mol/(kg • h).

9. The method as claimed in one of the preceding claims, **characterized in that**:
alcohols, in particular branched alcohols, preferably branched primary alcohols, and/or aldehydes and/or ketones and/or alkanes and/or aromatic compounds as well as mixtures thereof, are obtained as the product or product mixture, in particular wherein C₅-C₃₅ compounds, in particular C₅-C₃₀ compounds, preferably C₆-C₂₅ compounds, preferably C₆-C₂₀ compounds are obtained as the product or product mixture; and/or
during the reaction, water and optionally hydrogen, in particular water and hydrogen, are generated, in particular wherein at least one mole of water is generated per mole of compound (A) employed.

10. The method as claimed in one of the preceding claims, **characterized in that** the product or product mixture obtained subsequently undergoes a hydrogenation process step and/or a hydrotreatment process step, preferably a hydrotreatment process step.

## Revendications

1. Procédé, destiné à réaliser une condensation et/ou un couplage catalytique de composés organiques contenant au moins une fonction oxo et/ou hydroxyle avec des composés CH acides pour créer des alcools supérieurs, des aldéhydes, des cétones, des aromates et/ou des alcanes, ainsi que leurs mélanges, lors duquel, on met en oeuvre en tant que mélange d'éduit
(A) au moins un composé organique contenant au moins une fonction oxo et/ou hydroxyle, qui comporte au moins 3 atomes de carbone et qui est choisi dans le groupe des alcools primaires et/ou secondaires, des aldéhydes et des cétones ainsi que de leurs mélanges,
avec
(B) au moins un composé CH acide, qui comporte au moins 3 atomes de carbone, le composé CH acide (B) étant choisi dans le groupe des alcools primaires et/ou secondaires, des acides carboxyliques, des anhydrides d'acides carboxyliques, des esters d'acides carboxyliques, des aldéhydes, des cétones, des nitriles, des composés nitro, des nitrates organiques et de leurs mélanges,
(A) et (B) pouvant être identiques,
et on les fait réagir en présence d'au moins un catalyseur, le catalyseur comprenant un substrat de charbon actif, lequel est doté d'au moins un métal,
en tant que catalyseur étant mis en oeuvre un charbon actif façonné qui est susceptible d'être produit à l'aide d'un procédé de production de charbon actif façonné à partir d'un support de carbone, d'un agent liant et d'un composant catalytique de la formule générale (I)
[M]ₘ₃[AOₙ₄]ₘ₄ (I)
dans laquelle
- M désigne un cation et est choisi dans le groupe des cations de métaux alcalin ou alcalino-terreux ;
- m3 et m4 désignent des coefficients stoechiométriques entiers, avec m3 ≥ 1 et m4 ≥ 1 ;
- [AOₙ₄] désigne un anion oxygéné avec le coefficient stoechiométrique entier n4 ≥ 1 ;
- [AOₙ₄] est choisi de préférence dans le groupe des carbonates ou hydroxydes,
l'agent liant étant obtenu par la mise en réaction d'une matière de départ hydrosoluble contenant des hydrates de carbone avec une teneur en glucose de ≥ 50 % en poids, le support de carbone étant mélangé d'abord avec le composant catalytique, le mélange du composant catalytique et du support de carbone étant amalgamé ensuite avec l'agent liant, le mélange ainsi obtenu de support de carbone, de composant catalytique et d'agent liant étant ensuite compressé en ébauches et les ébauches étant carbonisées et activées, l'agent liant étant obtenu par la mise en réaction de la matière de départ contenant des hydrates de carbone avec un granulat, pour obtenir l'agent liant, le granulat étant ajouté à la matière de départ contenant des hydrates de carbone avant l'amalgame de l'agent liant avec le mélange du support de carbone et du composant catalytique, le granulat étant choisi dans le groupe des acides phosphoriques et/ou de leurs sels, des acides sulfuriques et/ou de leurs sels et/ou des dérivés d'acides sulfuriques et/ou de leurs sels.

2. Procédé selon la revendication 1, **caractérisé en ce que** le composé organique contenant au moins une fonction oxo et/ou hydroxyle (A) est choisi parmi des composés en C₃ à C₂₅, notamment des composés en C₃ à C₂₀, de préférence des composés en C₃ à C₁₅, de manière préférentielle, des composés en C₃ à C₁₀, de manière particulièrement préférentielle, des composés en C₃ à C₈, ainsi que des mélanges de différents composés avec le nombre d'atomes de carbone précédemment cité ; et/ou en ce que le composé organique contenant au moins une fonction oxo et/ou hydroxyle (A) est choisi parmi des composés linéaires ou ramifiés.

3. Procédé selon la revendication 1 ou 2, **caractérisé en ce que**
le composé CH acide (B) est choisi parmi des composés en C₃ à C₂₅, notamment des composés en C₃ à C₂₀, de préférence, des composés en C₃ à C₁₅, de manière préférentielle, des composés en C₃ à C₁₀, de manière particulièrement préférentielle, des composés en C₃ à C₈, ainsi que des mélanges de différents composés avec le nombre d'atomes de carbone précédemment cité ; et/ou en ce que le composé CH acide (B) est choisi dans le groupe des alcools primaires et/ou secondaires, des acides carboxyliques, des anhydrides d'acides carboxyliques, des esters d'acides carboxyliques, des aldéhydes, des cétones, des nitriles, des composés nitro, des nitrates organiques et de leurs mélanges, de préférence dans le groupe des alcools primaires et/ou secondaires, des aldéhydes, des cétones et de leurs mélanges.

4. Procédé selon l'une quelconque des revendications précédentes, **caractérisé en ce que** le composé CH acide (B) est choisi parmi
des alcools primaires et/ou secondaires de la formule générale (I) 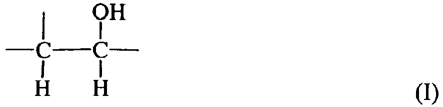 et/ou
des composés nitro et/ou des nitrates organiques de la formulé générale (II) 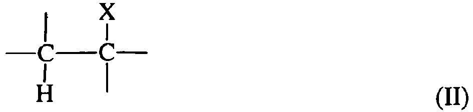 avec X = NO₂, ONO₂
et/ou
des composés carbonylés de la formule générale (III) 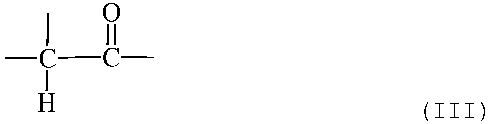 et/ou des composés nitrile de la formule générale (IV)

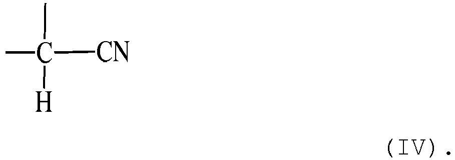

5. Procédé selon l'une quelconque des revendications précédentes, **caractérisé**
**en ce que** le procédé est réalisé dans la phase gazeuse et/ou dans la phase liquide et/ou dans la plage surcritique, de préférence dans la phase gazeuse ; et/ou en ce que le procédé est réalisé au-delà des températures d'ébullition des éduits et/ou des produits, de préférence au-delà des températures d'ébullition des éduits et des produits ; et/ou
**en ce que** le procédé est réalisé à des températures de l'ordre de 150 °C à 600 °C, notamment de 250 à 450 °C, de préférence de 300 à 400 °C ; et/ou
**en ce que** le procédé est réalisé sous pression réduite, sous pression atmosphérique ou sous pression élevée, de préférence sous pression atmosphérique ou sous pression élevée, notamment sous une pression absolue de l'ordre compris entre la pression atmosphérique et 150 bar, notamment de l'ordre compris entre la pression atmosphérique et 80 bar, de préférence dans l'ordre compris entre la pression atmosphérique et 25 bar.

6. Procédé selon l'une quelconque des revendications précédentes, **caractérisé en ce que** le procédé est réalisé avec des temps de réaction et/ou des temps de contact de l'ordre de 0,01 minute à 12 heures, notamment de 0,1 minute à 10 heures, de préférence de 1 minute à 5 heures, notamment le procédé étant réalisé dans la phase gazeuse et/ou dans la plage surcritique, avec des temps de réaction et/ou des temps de contact de l'ordre de 0,001 à 120 secondes, notamment de 0,01 à 60 secondes, de préférence de 0,05 à 30 secondes, et/ou notamment le procédé étant réalisé dans la phase liquide avec des temps de réaction et/ou des temps de contact de l'ordre de 0,01 minute à 12 heures, notamment de 0,1 minute à 10 heures, de préférence de 1 minute à 5 heures.

7. Procédé selon l'une quelconque des revendications précédentes, **caractérisé en ce que** le procédé est réalisé en présence d'eau, de préférence sous la forme de vapeur d'eau et/ou en présence d'hydrogène.

8. Procédé selon l'une quelconque des revendications précédentes, **caractérisé**
**en ce que** le procédé est réalisé avec un rendement espace/temps, indiqué en quantité de tous les produits formés par volume de catalyseur et par unité de temps de l'ordre de 10 à 3.000 g/(litre • h), notamment de 25 à 2.500 g/(litre • h), de préférence de 30 à 2.000 g/(litre • h), de manière particulièrement préférentielle de 50 à 1.500 g/(litre • h) ; et/ou
**en ce que** le procédé est réalisé avec une vitesse spatiale, indiquée en quantité de matière de tous les produits formés par masse de catalyseur et par unité de temps de l'ordre de 0,1 à 100 mole/(kg • h), notamment de 0,5 à 25 mole/(kg • h), de préférence de 1,0 à 20 mole/(kg • h), de manière particulièrement préféréntielle, de 1,25 à 18 mole/(kg • h), de manière tout particulièrement préférentielle, de 1,5 à 15 mole/(kg • h).

9. Procédé selon l'une quelconque des revendications précédentes, **caractérisé**
**en ce qu'**on obtient en tant que produit ou que mélange de produits des alcools, notamment réticulés, de préférence des alcools primaires réticulés, et/ou des aldéhydes et/ou des cétones et/ou des alcanes et/ou des aromates, ainsi que leurs mélanges, notamment en tant que produit ou que mélange de produits étant obtenus des composés en C₅ à C₃₅, particulièrement des composés en C₅ à C₃₀, de préférence des composés en C₆ à C₂₅, de préférence des composés en C₆ à C₂₀ ; et/ou
**en ce que** lors de la mise en réaction, on génère notamment de l'eau et le cas échéant de l'hydrogène, notamment de l'eau et de l'hydrogène, notamment au moins une mole d'eau étant générée par mole de composé (A) mis en oeuvre.

10. Procédé selon l'une quelconque des revendications précédentes, **caractérisé en ce qu'**on soumet par la suite le produit ou le mélange de produits obtenu à une étape de procédé d'hydratation et/ou à une étape de procédé d'hydrotraitement, de préférence à une étape de procédé d'hydrotraitement.
